# EUROPEAN PATENT APPLICATION

(11) **EP 3 702 969 A1**
(43) Date of publication of application: **02.09.2020**
(21) Application number: 19159261.7
(22) Date of filing: 26.02.2019
(51) Int. Cl.: G06K 17/00, G06K 19/07, G06Q 10/08, A61B 90/90, A61J 1/12, G06K 19/077

(54) **INTEGRATED LABELING OF MEDICAL SUPPLIES**

(71) Applicant: Wiegers Kornelis, 9636 TD Zuidbroek (NL)
(72) Inventor: Wiegers Kornelis, 9636 TD Zuidbroek (NL)
(74) Representative: Wensvoort, Gert

(57) **Abstract**

The invention provides method to survey handling of a medical or biological product said method comprising use of a label for labelling a container suitable for containing said material, said label provided with information suitable for identifying said material, said label also provided with a monitoring device, and a reader capable of providing a read-out of said monitoring device, wherein said monitoring device is provided with data suitable for identifying said material, and said monitoring device is capable of recording handling conditions that may be detrimental to the quality of said material, and said reader is capable of providing a warning signal when said handling conditions have been detrimental to the quality of said material. Such medical products may be blood and/or blood products and other bio-materials like cells, tissues or organs, antibodies, antigens, hormones, (recombinant) peptides or proteins, or pharmaceutical preparations such as drugs, vaccines, antibodies, hormones, proteins, peptides and small molecules. The invention also provides a label for labelling a container such as a blood bag comprising label information suitable for identifying a biological component, a monitoring device, such as an electronic article surveillance (EAS) device and optionally means through which tampering with said label is made evident.

## Description

The present invention relates generally to apparatus, labels and methods to surveyor monitor the administration, handling, labelling, transportation, prescription and administering of medical supplies. Such medical supplies may be blood and/or blood products and other bio-materials like cells, tissues or organs, antibodies, antigens, hormones, (recombinant) peptides or proteins, or pharmaceutical preparations such as drugs, vaccines, antibodies, hormones, proteins, peptides and small molecules. Apparatus, labels and methods presented herein employ electronically readable indicia to confirm the product characteristics, location, expiration date and identity and location of patients or caregivers every time such a biomaterial is handled. The present invention further relates to the collection, storage, communication, and location of information relevant to blood transfusions and other medical products such as pharmaceuticals, so that complete audit trails are recorded and guidance to the caregiver is provided in order to ensure that all required procedures are executed properly.

### Introduction

Distribution and wholesaling form part of the supply chain of medical products. Medical products often must be transported, handled and stored in a manner that mitigates the risk of exposure to temperatures outside labeled storage conditions; potentially impacting the safety, quality and effectiveness of the medical product. Pharmaceutical producers combine ingredients precisely, under specific conditions, while negotiating a maze of stringent regulations and quality controls. Companies that move or store medical supplies such as pharmaceutical products must meet similar demands. Many drugs are highly sensitive to temperature; some are extremely valuable; and all are subject to a complex array of government regulations. In the pharmaceutical supply chain, every detail counts.

To survey or monitor handling, labelling, transportation, and transfusion or admission of a very specific type of medical products, blood and/or blood products, pharmaceutical products, a quality system based on the principles of good practice must be introduced by blood establishments, i.e. pharmacy and hospital blood banks, establishing standards and specifications relating to this system. Pharmaceutical logistics is such a specialized discipline that drug manufacturers have long been reluctant to outsource to third-party logistics. In addition to requiring drug makers to ship their products across borders and over long distances, the pharmaceutical industry's global nature stymies manufacturers with a complicated array of regulations governing transporting drugs in different countries. Pharmaceutical industry service providers need to be aware of about 70 different sets of regulations. Environmental controls play a key role in maintaining drug safety, quality and efficacy. Temperature is one of the most important parameters to control. Drugs must be stored, and transported according to predetermined conditions (for example, temperature, etc.) as supported by stability data. Temperature excursions outside of their respective labeled storage conditions, for brief periods, may be acceptable provided stability data and scientific/technical justification exists demonstrating that product quality is not affected. One logistics function unique to the pharmaceutical industry is the work that supports clinical trials. Rather than distribute large quantities of a drug for use in the market at large, manufacturers conducting trials move product to labs and hospitals and, often, directly to patients' homes. The demand for shipments to clinical trial sites tends to ebb and flow, and each project is unique. Various studies go on at the same time, and all have different requirements.

In the mid-1990s to early 2000s - what we call the "early days" of temperature-controlled transport for pharmaceutical products - semi- active packaging solutions made from expanded polystyrene and expanded polyurethane ruled the cold supply chain. These simple (but often mighty) insulation systems, often referred to as "styrofoam" or "polystyrene", come in many sizes and consist of an outer shell of insulating material in a cardboard box and refrigerated or frozen coolants, such as water-based gel packs or dry ice. These packages are validated for a certain time period and shipping range - usually short-haul travel and shipments between +2°C and +8°C.

Polystyrene and polyurethane are commonly used today as an inexpensive option for shipping products short distances, such as between a distribution center and a treatment site. However, this low-tech solution is not right for every shipment. The packaging often experiences temperature excursions and requires different configurations for "summer" and "winter" climates, making it more susceptible to human error. And finally, polystyrene and polyurethane packaging cannot be reused and must be disposed of at the delivery site, making it a burden on the environment.

Storage conditions now should be defined and described on the label of the product. All drugs should be stored according to the conditions described on the label. When specified on the label, controls for humidity, light, etc., should be in place. Storage areas should be designed or adapted to ensure good storage conditions. In particular, they should be clean, dry, have adequate circulation and maintained within acceptable temperature limits.

The label should specify any special storage conditions required for the product. Adherence to these conditions should be checked, monitored and recorded. Temperatures should be controlled and monitored using calibrated monitoring devices and records of temperature and alarms, where applicable, should be maintained. Monitoring of storage facilities is conducted at points representing the worst case scenarios of the temperature range based on temperature mapping.

More can be read in "Parenteral Drug Association (PDA), Cold Chain Guidance for Medicinal Products - Maintaining the Quality of Temperature-Sensitive Medicinal Products through the Transportation Environment, Technical Report 39, PDA Journal of Pharmaceutical Science and Technology, Volume 61, Issue No. S-2, 2007."Every pharmaceutical product must be checked against a database to prevent falsification (2011 Falsification Medical Directive, EU-FMD)

With regard to documentation and record-keeping, blood establishments must keep the following up to date: documentation on operational procedures, guidelines, training and reference manuals, and reporting forms; records of activity and basic testing requirements for whole blood and components thereof;

Records of applications made by establishments for accreditation, provisions relating to existing establishments and notifications of any serious adverse reactions or events.

With regards to haemovigilance, the traceability of blood and blood components, from donor to recipient and vice versa, must be guaranteed. In order to ensure full traceability, blood establishments and hospital blood banks must implement a system permitting unmistakable identification of each single blood donation and each unit of blood and components thereof.

According to Implementing Directive 2002/98/EC of the European Parliament and of the Council as regards traceability requirements and notification of serious adverse reactions and events
http://eur-lex.europa.eu/legal-content/EN/TXT/PDF/?uri=CELEX:32005L0061&from=NL

A tracking and tracing system must me implemented by all EU member States and a system permitting an equivalent level of traceability must also be operated with regard to blood and blood components imported from third countries. Any serious adverse events must be notified to the competent authority. Blood establishments and hospital blood banks must have in place a procedure for the efficient withdrawal from distribution of blood or blood components associated with a serious adverse event and conditions relating to donations and donors in order to ensure the quality and safety of blood and blood components must be set. Donors must be provided with certain information when giving blood. This includes information on the characteristics of blood and blood products, and an undertaking by the blood collection center to contact the donor if the results of the tests reveal any form of pathology. Certain information must be obtained from donors, for example their identification, their state of health and medical history, and a signature confirming that the donor has read and understood the information provided. The donor is subject to an evaluation procedure and must satisfy the criteria concerning physical suitability, such as age, and donation criteria, such as the time interval between donations. Permanent deferral criteria are also drawn up in order to protect both the donor and the recipient (for example in case of diseases that could place either the donor or the recipient in danger). An examination of the donor, including an interview, shall be carried out before any donation of blood or blood components. Each donation of blood or blood components must undergo certain tests.

### Definitions used herein.

Blood component: a constituent of blood (red cells, white cells, platelets, plasma) that can be prepared by various methods (centrifugation, filtration and freezing).

Hospital blood bank: a hospital unit which stores and distributes and may perform compatibility tests on blood and blood components exclusively for use within hospital facilities, including hospital based transfusion activities.

Distribution: the act of delivery of blood and blood components to other blood establishments, hospital blood banks and manufacturers of blood and plasma derived products. It does not include the issuing of blood or blood components for transfusion. Blood establishment: any structure or body that is involved in any aspect of the collection and testing of human blood or blood components, whatever their intended purpose, and their processing, storage, and distribution when intended for transfusion.

Haemovigilance: a set of organized surveillance procedures relating to serious adverse or unexpected events or reactions in donors or recipients, and the epidemiological follow-up of donors.

Serious adverse event: any untoward occurrence associated with the collection, testing, processing, storage and distribution of blood and blood components that might lead to death or life-threatening, disabling or incapacitating conditions for patients or which results in, or prolongs, hospitalization or morbidity.
Blood product: any product derived from human blood or plasma.

Blood: whole blood collected from a donor and processed either for transfusion or for further manufacturing.

Active RFID systems use self-powered RFID tags that continuously broadcast their own signal. Active RFID tags are commonly used as "beacons" to accurately track the real-time location of assets or in highspeed environments such as tolling. Active tags provide a much longer read range than passive tags, but they are generally also much more expensive. Passive RFID systems use tags that are powered by the electromagnetic energy transmitted from an RFID reader. Passive RFID tags have applications including access control, file tracking, race timing, supply chain management, smart labels, and more. The lower price point per tag make employing passive RFID systems economical for many industries.

Blood donations save lives. But it does happen that donations become mixed up en route from the donor to the recipient, or the blood deteriorates because it has not been sufficiently cooled or are cooled too much- things that are very difficult to verify before a transfusion takes place. The blood is collected from a donor into a container, herein and generally called a blood bag, this may be done outside the blood bank, were there are no controlled conditions for storage. The blood can also be taken from the donor in the blood bank where conditions are more or less controlled. After collection, blood is identified with its donor identification (ID) and stored in an information system together with some samples of the blood to check later whether there would be problems with the donated blood at the patient. The full blood from the donor is often split up into: platelets, red blood cells and plasma, after centrifugation. The separate components are again stored in a containers or blood bags which are again labeled with blood bank identification, blood component type and donor information. Platelets are often combined from multiple (i.e. 5-6) different donors into 1 new container or blood bag and the donor ID (identification) of these multiple donors together with the component ID's are stored in an information system. Blood bank information, donor ID and additional information, such as component type and expiration date are printed on the blood bag label. If special treatments have been performed with these blood components, this may also be printed on the label and stored in the blood bank information system. Blood transfusions are usually performed at hospitals. Hospital blood banks receive blood from the blood supply agency, perform any tests they may require to assure the type and quality of the blood and place the blood into the blood bank stock. The first step in the transfusion process is testing of the patient's blood. This requires that a blood sample be drawn from the patient, correctly labelled with the patient's identification, and sent to the blood laboratory. The laboratory tests the patient's blood to determine the correct blood type for the patient, and any special requirements the patient may have. Once these factors are known, a suitable blood unit is retrieved from the blood bank stock and is labelled as suitable for the particular patient. The designated blood unit is placed into a storage location until it is needed. The blood laboratory keeps detailed records of the testing of the blood unit and the patient's blood. The detailed record of the patient's blood test results, blood type and requirements is usually stored in a blood bank computer system (McKesson Corporation, www.mckesson.com, Misys, www.misys.com, Meditech, www.meditech.com and many others). This information, along with a history of previous blood transfusions, may be used to quickly allocate additional blood units for the patient without the need to repeat the original blood test, in a process call 'electronic issue' of blood units. When the patient requires blood for transfusion, someone is sent to the blood bank to collect the prepared blood unit. They are expected to ensure that they have collected the correct blood unit, and to record the time that the blood unit was retrieved. Accepted practice requires that blood that has remained outside of refrigeration for more than 30 minutes should not be transfused. It is the responsibility of the person collecting the blood from the blood bank to ensure that it is promptly delivered. Reducing medical errors and enhancing the safety and quality of healthcare delivery is widely recognized as a key priority. In this regard, enabling error-free delivery of blood products is an essential component of enhancing overall healthcare safety. While the general public associates blood transfusion risk with exposure to infectious hazards, mis-transfusion (mismatch between patient and blood) is the most prevalent serious hazard of transfusion. (Data about transfusion reactions is collected and reported and is called: haemovigilance). The use of radio-frequency identification device (RFID) technology in blood banks and transfusion medicine has the potential to improve operational efficiency and advance patient safety at point of care by automatically identifying, reconciling, and tracking blood products throughout the blood supply chain. ISO/IEC 18000-3 mode 1 standard 13.56 MHz RFID tags have been accepted by the International Society for Blood Transfusion (ISBT) and the United States Food and Drug Administration (FDA) as data carriers to integrate with and augment ISBT 128 barcode data carried on blood products. The use of 13.56 MHz RFID carrying ISBT 128 data structures allows the global deployment and use of RFID, supporting both international transfer of blood and international disaster relief. With such a blood-tracking system, hospital workers attach a self-adhesive 1.5-by-1-inch RFID tag to each bag of blood arriving at the hospital. The tag's passive 13.56 MHz RFID chip has 2 kilobytes of memory for storing a unique number, the hospital tracking number (used by the blood bank system) and information on blood type. These numbers and data are also saved in a secure database containing details about the blood's origin, its designated purpose and, once dispensed, its recipient. When a nurse wants to prepare a blood transfusion, he or she uses a handheld computer with an RFID reader or interrogator to read the data encoded on the blood bag's RFID chip and the inlay embedded in the patient's ID bracelet. The latter includes a patient ID number and such medical information as the patient's weight and blood type. The data from the patient and the bag must match before the blood can be transfused to the patient, minimizing the risk of patients receiving the wrong type of blood and/or if the allowed temperature limits are not exceeded. US patent application 11/795,677 provides a method and a system for using RFID (Radio Frequency Identification) in the workflow of a blood center and a medical institution from a network Customer Relationship Management (CRM) information system. The CRM comprises an RFID technique system, a computer database system, and a computer information network. The information transferred between an integrated circuit and a reader-writer occurs through the radio waves in the workflow of, for example, a blood center or other medical institution. The related information ranges from identity information to biological product information. In each procedure of the blood collecting and supply workflow, the information is read/written by the computer into electronic label and through the computer information network into the service management information system. The work flow of blood centers (stations), such as blood test reports before and after blood transfusions and the desired information of people and objects involved in blood transfusions, is thereby recorded by the RFID system. There are products that attempt to ensure that blood samples drawn from a patient for testing are correctly labelled. (e.g., Safe Track, DataLog International Ltd., www.dataloguk.com, BDID, Becton Dickinson Ltd, www.bd.com, McKesson Corporation, www.mckesson.com) These systems do a good job of making sure that the label applied to the blood sample collected from the patient match the information on the patient's wristband, but do not offer any improvement in the completion of the audit trail for the complete transfusion process. A consortium led by Siemens, in conjunction with the Medical University in Graz, Austria, has developed an RFID system with temperature sensors. The chip or radiolabel contains the blood data and monitors the cooling chain with a temperature sensor. The chip is not removed at any point during the entire chain from donor to patient- even not in the centrifuge. This electronic system comprising a temperature sensor and chip was considered to be so robust that it would be able to withstand being centrifuged at up to 5,000 times the acceleration of gravity. Shortly before the donation, a reader is used to read the chip data and check its accuracy. This system was meant to be the first to enable a complete chain of documentation to be maintained from the donor to the recipient, significantly increasing patient safety, however, further evaluation studies of this system demonstrated a distinct lack of robustness when centrifuged and/or pasteurized under practical conditions of blood bank use.

There have also been attempts to improve the monitoring of the movement of blood units from place to place, to ensure that the blood is correctly stored, that all movements are recorded and that the blood is not outside of refrigeration for more than the allowed time. (e.g., Blood Track, Data Log International Ltd., www.dataloguk.com). These systems provide valuable audit information for movements from one storage location to another, but loses track of the blood unit in the critical last step, when the blood unit is removed for transfusion. In addition, the systems rely on users to scan various barcodes in the correct order to ensure that the movement of the blood units is correctly recorded.

There have also been attempts to improve the transfusion process itself. There are products that use barcode scanners to compare bar-coded information on the patient's wristband, the compatibility label and the blood unit to ensure a correct match. (e.g., Safe Track, DataLog International Ltd, www.dataloguk.com, Itrac, Immucor, www.immucor.com). These products do provide a means for improving the safety of the transfusion step, but do not return information to the blood bank to confirm the completion of the transfusion or report reactions. They also fail to provide a means to ensure that the blood unit to be transfused has been stored and transported correctly and within the acceptable time limits. The European regulations 2002/98/EG and 2001/83/EG are currently not met because it is considered technically not possible yet to fully check the quality of blood components over the whole ranges of time. Managers in hospitals now 'assume' that the required safe temperature limits are met because of the cooling systems they use, but with our objective measurements during three pilots: this turns out not to be the case! The warming up of blood components is caused by taking them out of the cooling systems at several transfer moments: labeling, cross matching and AB0 screening before admitting the blood to the patient. There is a worldwide rule that blood should be admitted within 30 minutes after it is taken out of the refrigerator, but this rule is generally not met and cannot be checked at the moment! Some hospitals have protocols that within an hour, a returned blood bag can be reused. All these steps causing warming up, which can add up to a couple of hours in the complete logistic chain, resulting in deterioration of the quality of the blood cells and increased growth of bacteria. Deterioration of blood also may cause an increased concentration of iron in the patient's blood, which is poisoning. Thus, there remains a need in the art for improved systems, apparatus and methods for ensuring reliable transfusion of blood and/or blood products into a specified patient.

### The invention

The invention provides method to survey handling of a medical or biological product said method comprising use of a label for labelling a container suitable for containing said material, said label provided with information suitable for identifying said material, said label also provided with a monitoring device, and a reader capable of providing a read-out of said monitoring device, wherein said monitoring device is provided with data suitable for identifying said material, and said monitoring device is capable of recording handling conditions that may be detrimental to the quality of said material, and said reader is capable of providing a warning signal when said handling conditions have been detrimental to the quality of said material. Such medical products may be blood and/or blood products and other bio-materials like cells, tissues or organs, antibodies, antigens, hormones, (recombinant) peptides or proteins, or pharmaceutical preparations such as drugs, vaccines, antibodies, hormones, proteins, peptides and small molecules. The invention also provides a label for labelling a container such as a blood bag comprising label information suitable for identifying a biological component, a monitoring device, such as an electronic article surveillance (EAS) device and optionally means through which tampering with said label is made evident. The invention also provides a system to track and trace a medical product, such as a blood product, a cell or cells, a tissue or an organ, comprising using a label according to the invention to label the container in which said product is stored or transported. The invention also provides a system according to the invention comprising using at least a second label after a processing step in the processing of said biological product and reading data from the log file of a first integrated label and importing said date into the log file of said second integrated label. The invention provides such method and label and system to prevent a receptor, such as a patient, from receiving a medical product from a non-intended or wrong source, a medical product that has not been handled well and/or to collect haemovigilance data.

The invention provides a method to survey or monitoring handling of a medical or biological product or material, said handling preferably comprising labelling, storing, transportation, freezing, thawing, heating, (therapeutic) application or use of a medical or biological product or material like a medicine, a drug, a vaccine, an hormone, a tissue or organ, a (recombinant or synthetic) nucleotide, peptide, protein, replicon, cell or organism, an antibody, antigen, hormone, drug, blood and/or blood product or transfusion of blood, blood component and/or blood product comprising use of
a label for labelling a container with such material, such as a blood bag, comprising label information suitable for identifying said medical product or biological material, said label provided with a monitoring device, such as an electronic article surveillance (EAS) device, preferably wherein said EAS device is a radio-frequency identification (RFID) device, and a reader capable of providing a read-out of said monitoring device, preferably wherein said reader is an EAS reader, more preferably wherein said reader is an RFID reader; wherein said monitoring device is provided with data suitable for identifying said biological material or aspects thereof such (depending on the nature of the material) as name, source identification, donor identification, production date, production location, blood or blood component identification, unique identification number, results of (virological, bacteriological, serological or nucleic acid) testing of said material, blood group, collection date, expiration date, intended use, and other desired information to allow monitoring of haemo(vigilance) data, handling and storage conditions such as temperature, time, transport, location. It is preferred that said reader is capable of providing date and/or time and /or location and /r identity of the staff member taking care of reading, providing a warning and/or exclusion signal with regards to time or temperature, providing a determination of location, providing a report, providing batch wise or multiple monitoring. Medical products as used herein may be blood and/or blood products and other biological products like cells, tissues or organs, antibodies, antigens, hormones, (recombinant) peptides or proteins, or pharmaceutical preparations such as drugs, vaccines, antibodies, hormones, proteins, peptides and small molecules.

Typically the invention provides a method to survey of handling of a medical or biological product or material comprising use of
a label for labelling a container suitable for containing said material, said label provided with information suitable for identifying said material, said label also provided with a monitoring device, and
a reader capable of providing a read-out of said monitoring device,
wherein
said monitoring device is provided with data suitable for identifying said medical or biological product or material, and
said monitoring device is capable of recording handling conditions that may be detrimental to the quality of said material, and
said reader is capable of providing a warning signal when said handling conditions have been detrimental to the quality of said material, wherein said monitoring device comprises an electronic article surveillance (EAS) device and said reader is an EAS reader, preferably wherein said monitoring device is a radio-frequency identification (RFID) device, preferably an active RFID device, and wherein said reader is an RFID reader, preferably also said reader is capable of reading a barcode. It is preferred to also use a writer capable of writing data in the monitoring device, preferably wherein said reader is also capable of writing or storing said data from said read-out in said monitoring device and/or in said reader and/or in an external data storing device such as an hard disk or a system of hard disks, preferably wherein said storing device is a cloud-based device. To best monitor handling conditions, it is preferred that said monitoring device is capable of recording temperature and/or time. Furthermore, it is preferred that said reader is capable of recording date of and/or time of and/or location of and/or identify a person taking part in providing said read-out. In a most preferred embodiment, said reader is capable of recording read-outs of at least two, preferably at least four, more preferably at least 10 monitoring devices that are simultaneously submitted to said reader. In a preferred embodiment of the invention a method is provided wherein said biological material comprises blood, a blood component or a blood product.

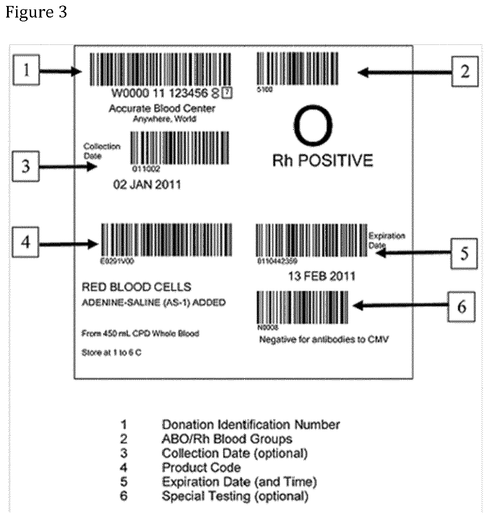

The present invention also relates generally to apparatus, labels and methods for monitoring the handling, labelling, storing, transportation, and transfusion of blood and/or blood products and bio-materials like cell, tissues or organs. Apparatus, labels and methods presented herein employ electronically readable indicia to confirm the identity and location of patients, blood units, tissue, and caregivers every time a blood unit, tissue or organ is handled. The present invention further relates to the collection, storage, and communication of information relevant to transfusions (Haemovigilance) such that complete audit trails are recorded and guidance to the caregiver is provided in order to ensure that all required procedures are executed properly. In a preferred embodiment, the invention provides a system and its components for quality control of blood in blood bags. The invention provides an label for labelling a container such as a blood bag comprising label information suitable for identifying a biological component, a monitoring device, such as an electronic article surveillance (EAS) device. It is preferred that said label information comprises information encoded with a barcode such as indicated in figure 3 when to identify biological component as a blood or a blood component, said component preferably selected from the group of plasma, platelets or red blood cells. In a preferred embodiment said EAS device is a radio-frequency identification (RFID) device as defined herein below, preferably wherein said EAS device is provided with an antenna and/or power source. In a much-preferred embodiment, the invention provides a label wherein tampering with said label results in abolishing the function of said EAS device, such as further identified herein below. Preferably, said tampering results in disrupting a connection with an antenna or power source of said device. The invention typically provides a label wherein said monitoring device allows for temperature sensing of the content of said container and/or wherein said monitoring device allows for tracking and tracing said container. Herewith the invention provides a method to control quality of a biological product, such as a blood product, a cell or cells, a tissue or an organ, comprising using an integrated label according to the invention to label the container in which said product is stored or transported. Also, a method is provided to track and trace a biological product, such as a blood product, a cell or cells, a tissue or an organ, comprising using an integrated label according to the invention to label the container in which said product is stored or transported. In its most preferred form, the invention provides a method for providing a chain of documentation arising from the chain of events from the donation of donor blood to the transfusion of a blood product to a recipient comprising labeling a container comprising blood or a blood component with an integrated label according to the invention.

The invention also provides a method and system for quality control of a medical or biological product or material such as pharmaceutical preparation or medicine such as drug, a vaccine an hormone, an antibody or such as blood or a blood component stored in a blood bag, comprising using at least a second integrated label in the production chain from donor to transfusion, preferably after a processing step in the processing of said medical or biological product of blood or blood component, preferably wherein said processing step comprises centrifugation or splitting of a blood product. The container containing the blood product is than re-labelled or re-stickered with said all least second integrated label. The data of the first label is then electronically transferred to the second (new) label to transfer all the data from the original label to the new label(s). In this relabeling routine as is provided herein it is a distinct advantage when the old or earlier applied label can simply be invalidated abolishing the function of the EAS device, preferably of the RFID device, by deliberately tampering with it, or electronically erase the data from the original label In this way, the invention provides a system to survey or control quality of a biological product, such as a blood product, a cell or cells, a tissue or an organ, comprising using a sequence of integrated labels (at least 2) according to the invention to label the container(s) in which said product is subsequently stored or transported and a system to track and trace a biological product, such as a blood product, a cell or cells, a tissue or an organ, comprising using an integrated label according to the invention to label the container in which said product is stored or transported. The invention also provides a system comprising using at least a second integrated label after a processing step in the processing of said biological product and reading data from the log file of a first integrated label and importing said date into the log file of said second integrated label, herein also called "re-stickering", preferably wherein said processing step comprises centrifugation or splitting of a blood product. The invention also provides a container such as a blood bag provided with an integrated label according to the invention.

In a one embodiment, the invention provides a system for quality control of blood or blood products or blood components, hereinafter called blood, in blood bags or other blood containers, hereinafter called blood bags, the system comprising blood bags provided with or attached to an integrated label, which includes a blood temperature sensor and label internal control means for measuring periodically the temperature of the blood inside the blood bag and for storing the measured temperatures in a log file inside the integrated label. In the label also is stored all the information about the blood component such as: donor ID, expiration date, component type etc., so it can also be used as transfusion control system when admitting blood to a transfusion patient. It is preferred that said label is tamper-evident, as provided herein. In another embodiment, the invention provides a system for quality control of pharmaceutical preparation, or drug, or vaccine or toxin (see also Table 1 herein) hereinafter called medicine, in container, the system comprising a container or a label on the syringe or medication provided with or attached to an integrated label, which includes a temperature sensor and label internal control means for measuring periodically the temperature of the medicine inside the container or syringe and for storing the measured temperatures in a log file inside the integrated label and determine the location of the product. In the label also is stored all the information about the medicine such as: donor ID, expiration date, component type etc., so it can also be used as medicine control system when administering it to a patient. It is preferred that said label is tamper-evident, as provided herein. In a further embodiment, the invention provides application of a tamper-evident label (herein also called an integrated label) allowing identification for automatic identification, preferably radio-frequency identification (RFID), tracking, temperature sensing and status-monitoring of medicines or blood and blood products across all entities in the supply chain - from the point of collection, through pharmaceutical product or blood product manufacturing and distribution, to delivery of a product by a healthcare provider to a patient. The invention provides a tamper-evident, preferably RFID-enabled, product tracking solution (to complement or replace bar-coding) that overcomes associated technical and policy environment challenges and complements good manufacturing practices using a quality systems approach. As used herein, an RFID device or label is an object that can be applied to or incorporated into a product, animal, or person for the purpose of identification using electromagnetic radiation that lies between audible and infrared radiation. An RFID label contains at least two parts. One part is an integrated circuit for storing and processing information, modulating and demodulating a (RF) signal and can also be used for other specialized functions. The second part is an antenna for receiving and transmitting the signal, which can be read from several meters away. RFID circuitry is well known to those skilled in the art and may be provided in a plurality of configurations, for example, passive and active. A passive label requires no internal power source and depends for activation and operating power upon the signal emitted by a reader, whereas an active RFID label includes an internal power source, such as a battery or a photovoltaic cell, which is used to power the integrated circuits and broadcast the signal to the reader. An active label typically has a much longer range (approximately 15 m) and larger memory than a passive label, as well as the ability to store additional information. In a preferred embodiment, the invention provides an integrated label made from tamper evident or tamper-indicating material designed to prevent access to the embedded tag, RFID or RF transponder without noticeable visible damage to the label. This unique security feature deters the removal, alteration, replacement, or transference of the embedded electronic article surveillance (EAS) device, RFID or RF transponder. Tamper evident label materials are engineered to self-destruct when removed from a substrate. The primary function of these products is to produce a tamper-indicating label or seal by causing the label to fracture when removal from a substrate is attempted. There are a variety of different tamper evident or tamper-indicating label materials available from 3M Company, St. Paul, Minn., including 7610 ScotchMark Destructible White Vinyl. The 7610 product uses a fragile cast vinyl face with very low tensile and tear resistance designed to crack and break when peeled off of a surface because the permanent adhesive bond is stronger than the label face. Other types of tamper-indicating labels utilize a "void" pattern in the adhesive as with 3M 7866 Polyester. This product uses a clear polyester face stock with a white-pigmented adhesive. A silicone type pattern is printed on back of the label face such that as the label is peeled from a surface only part of the adhesive removes with the label. The adhesive pattern is such that it creates a "VOID" word pattern across the label face stock. Applications for destructible tamper evident labels include safety warning labels, warranty seals, packaging seals, license labels, calibration seals, and asset labels. One embodiment of the invention uses a label or label material M1 made from a self-destructible material such as the 3M 7900, 7930 or 7610 ScotchMark Destructible White Vinyl or similar tamper-indicating materials designed to fracture or break apart when peeled up from a substrate. This label stock is printable on demand with thermal transfer ribbon ink, or it can be preprinted on a press using flexographic, letterpress, offset, rotogravure, screen, or other technology. This label with adhesive and release liner contains an embedded RFID transponder tag, preferably an active RFID transponder tag, inserted such that it is sandwiched between the adhesive and the release liner. After printing, the label is removed from the release liner, which protects the label during printing and before application; the label or transponder remains attached to the adhesive and is applied with the label to a blood or blood product bag. A further embodiment of a tamper evident integrated label according to the invention uses "3M 7847 Laser Markable Tamper Evident Label Material" or similar material with an embedded label or transponder. This is a specialty film that can be imaged and "kiss cut" by a laser beam. The top layer can be ablated by a laser beam to create an image (top face layer is a black acrylate, bottom face layer is a white acrylate). This engraved inscription provides long-term readability and abrasion resistance. The destructible face stock material provides tamper evidence to meet security labeling requirements. A third embodiment for providing tamper evidence is to incorporate a "void" adhesive pattern into the label product. Materials such as 3M 7866 Gloss White Polyester, 7389 Silver Polyester, and 7385 Tamper-Indicating Label Material for Dot Matrix Impact Printing are designed to provide a "void" or other message in the face stock when removal is attempted. The primary function of these label products is to create a non-transferable (non-reusable) label or seal by causing the "VOID" destruct message to appear in the label face when removed from a substrate. These label materials are manufactured by 3M using a clear polyester face stock with a pigmented adhesive. A silicone type "void" pattern is printed on back of the label face so that as the label is peeled from a surface only part of the adhesive removes with the label. The adhesive pattern is such that it creates a repeating pattern of the word "VOID" across the label face. The remaining adhesive on the substrate also displays the same "VOID" pattern. Any desired pattern, symbol or phrase may be applied in place of "VOID". Note, any of the tamper evident labels described above could be applied to a blood bag independently of the label or transponder (the label or transponder does not have to be embedded within the label). This can be accomplished simply by holding the label or transponder against the outer surface of the blood bag or adhesive of the label, then applying any label made of a tamper evident material over the label or transponder. In another embodiment, in addition to using the tamper evident label materials to prevent a label or transponder from being removed, altered, replaced, or transferred to another label or substrate, the label or transponder itself may be formed in a tamper evident manner. RFID transponders are typically manufactured using a polyester or polyamide base film designed to accept the printed antenna and mounted integrated circuit chip and optionally the power source. A tamper-evident transponder is manufactured by using a tamper evident material as the transponder base film. Also, the RFID transponder substrate can be processed to include propagation tear cuts around the perimeter of the antenna base film or around the power source base film. When the attempt is made to remove the tag or transponder from either the label or a blood bag surface the label is attached to, these propagation cuts will use the tensile strain created within the RF transponder through the removal process to sever the transponder into one or more pieces, thus destroying the antenna or power source connection to the chip and thereby the functionality of the label or transponder. An additional mechanism that could be added as an indicator of tampering would be to apply a covert technology such as holograms or micro-printing for authenticity on any of these integrated label designs. This would provide a secondary security measure to make it difficult to change or duplicate or re-use a smart label. Note also that these designs should not be limited to just RFID transponders. Any of these tamper-evident designs may also be applied to smart labels containing standard EAS devices (single bit electronic article surveillance devices). There may also be situations where the RF transponder or EAS device could be applied to the surface of a label instead of embedded in or under the label. This design may be necessary to readily identify if the RF transponder or EAS device is still present and has not been removed, altered, or tampered with in any manner. One aim of the present invention is to provide a substantial increase of the quality of blood and blood components or ingredients (blood plasma, blood platelets or red blood cells) in the chain of production, distribution, processing, administration (transfusion) of blood and blood components using blood bags or comparable blood containers, providing improved quality check means to be used at several locations in the chain or network. Another aim is to provide a substantial waste reduction in transfusion blood and blood components and thus to provide a substantial cost reduction. In a preferred embodiment, the invention provides a label additionally comprising checking means arranged for checking the measured blood temperatures against at least one threshold value and for producing an alarm code when the measured blood temperature transgressed the at least one threshold value. To meet those aims the invention provides a system for quality control of blood or blood components, hereinafter, for brevity's sake, called blood, in blood bags or other blood containers, hereinafter, for brevity's sake, called blood bags, the system comprising blood bags provided with or attached to an active temperature monitoring label, hereinafter called integrated label, which includes a blood temperature sensor and label internal control means for measuring periodically (e.g. once per 5 minutes) the temperature of the blood inside the blood bag and for storing the measured temperatures in a log file inside the integrated label, or by storing the exceeded threshold temperature, data en time in the integrated label. By measuring periodically the temperature of the blood or blood components inside the blood bag and storing the measured temperatures in a log file inside the or the exceeds thresholds, the logged course of the temperature of the blood inside the blood bag can be monitored and checked at several nodes in the production, distribution, processing and administration (transfusion) chain or network thus providing for improved means for a blood quality management system. It is noted here that the invention provides improved capabilities compared with the prior art, viz. by the invention's capability to monitor the blood temperature or the temperature of a pharmaceutical product automatically at short measuring periods, in all stages, from the begin to the end, of the location, production, distribution, processing and administration chain or network, hereinafter called production chain for brevity's sake, and not -as has been the standard practice until now- only at stages or nodes in the process chain or network where humans (*have to*) check the blood temperature manually, e.g. before a transfusion. It is known that blood and blood components or pharmaceutical products only are safe when they -during the complete production chain, from the beginning to the end- when their temperature did not transgress one or more security limits or thresholds. Whenever in the production chain (during its whole time span, not only at certain nodes in the production chain, including during transportation stages between the nodes) such security threshold has been transgressed, the quality of the blood or blood components or pharmaceutical products must be deemed to be insufficient and have to be removed from the production chain. To meet the requirement that the temperature of the blood product may not, at any moment, transgress its security/quality threshold(s), it is preferred that the system according to the invention, moreover, comprises checking means arranged for checking the measured blood temperatures or pharmaceutical products against at least two threshold values and for producing an alarm code when the measured (blood) temperature transgressed the at least one threshold value. It is noted here that the security temperature thresholds (limits) and max. shelf life values may have different values for the various (blood) products, viz. (on average). Preferably, the checking means are located in the integrated label. In a further preferred embodiment, the invention provides a system according to the invention, wherein the integrated label comprises wired, NFC or wireless communication means or electronic article surveillance devices arranged to communicate with label external control and/or reading means, hereinafter called label external control/reading means, provided with corresponding communication means, preferably wherein the possible alarm codes are stored in the log file of the integrated label, and preferably wherein the wireless communication means are RFID based. It is also provided that the label external control/reading means are arranged to read the temperatures and/or alarm codes stored in the log file of the integrated label, preferably wherein the label external control/reading means are arranged to set and/or adjust an at least one threshold value. The checking means may, however, also (instead of or additionally) be located in external means, as will be mentioned hereafter. The possible alarm codes (threshold transgression codes) may be stored in the log file of the integrated label. It may be preferred that the monitoring label comprises wired, NFC (Near Field Communication) or wireless communication means arranged to communicate with label external control and/or reading means, hereinafter called label external control/reading means, provided with corresponding, suitable communication means. Preferably, the wireless communication means are RFID based (classifying the monitoring integrated label as an RFID device). The label external control/reading means may be arranged to read the temperatures and/or alarm codes stored in the log file of the integrated label. Besides, the label external control/reading means may be arranged to set and/or adjust the at least one threshold value. Further, the label external control/reading means may be arranged for transferring the blood temperatures and/or alarm codes read from the log file to label external processing means for further processing, logging, analyzing, displaying etc. As already indicated in the preceding, the checking means may be located in the label external control/reading means and/or the processing means, independently of whether the monitoring label is (already) provided with such checking means. Where the blood bags are fit for the transportation of blood, blood plasma, blood platelets or red blood cells etc., It is preferred if the monitoring label comprises means for storing a content code identifying the blood bag's content or intended content. The label internal control means or label external control/reading means may be arranged then to set or adjust the at least one threshold value of the checking means in accordance with that content code defining the secure temperature area for the relevant bag content. As outlined in the preceding, the system according to invention is arranged for use in a chain or network for the production, distribution, processing, transportation, storage, transfusion etc. of blood using blood bags, comprising quality check nodes at several locations in the chain or network, the nodes being provided with quality checking stations, connected to a blood quality management system. In each chain node the quality of the blood bag content can be tested before further processing. The quality is always, at each node of the production chain, determined over the complete preceding time interval from the start point of the blood production (blood donation). Whenever in that interval a temperature limit transgression might have occurred, resulting in at least one alarm code, the quality check will result into rejection of the content of the blood bag and thus removal it from the production chain. In this way the blood quality is more reliable throughout the whole production chain, resulting in a much more reliable blood product at the chain's outlet, resulting in much safer surgery results. On the other side, less blood products are wasted, all blood products having unambiguously sufficient quality may pass in the production chain and be used in the surgery, while only blood products having unambiguously insufficient quality, are rejected and removed from the production chain. In this way application of the system according to the invention will result into less risk due to unambiguous quality/security control of the blood products, as well as less waste, as only blood products which are unambiguously justified as having bad or even dangerous quality, are removed. Awareness of these events may also be used to improve the quality in the production chain.

Although it is possible to check, in checking stations at the production chain nodes, to check the (blood or pharmaceutical product) quality by optical inspection of the e.g. optically perceivable alarm notification only by human operators, who could input their results into a quality management system (e.g. database), it will be preferred to use label external control and/or processing means which are part of said checking stations, thus enabling a regular flow of quality data which can be logged, processed and evaluated in any sophisticated, possibly distributed (e.g. via the Internet), computerized blood products quality system. During the whole lifetime of a blood product sample residing in the blood bag the sample's temperature is continuously (i.e. at certain short intervals) measured and compared with the relevant security thresholds. After any transgression an alarm code is written into the log file, which will detected at the next node, resulting in removal of the blood bag from the production chain/network. In a preferred embodiment, the invention provides a system for quality control of blood or pharmaceuticals additionally comprising checking means arranged for checking the measured temperatures against at least one threshold value and for producing an alarm code when the measured blood temperature transgressed the at least one threshold value wherein the checking means are located in the integrated label. The monitoring label NFC or wireless communication means or electronic article surveillance device is arranged to communicate with label external control/reading means, residing in the label terminals, which are also provided with such communication means. It is preferred to use RFID based communication means, by which the label is capable to exchange data and settings with the label terminals. In that way the label terminals or readers are suitable to read the temperatures and/or alarm codes stored in the log file of the monitoring label. Besides, the label terminals are capable to set and/or adjust the threshold values in dependency to the actual blood bag content. The label terminals are arranged for transferring the recorded blood temperatures and possible alarm codes read from the log file residing in the monitoring label to label external processing means for further processing, logging, analyzing, displaying etc. which label external processing means may reside in the servers or processing module. The blood bags may be fit for the transportation of blood, blood plasma, blood platelets or red blood cells etc., and the integrated label is capable to store, in its memory, a content code identifying the blood bag's content or intended content like pharmaceuticals. The label readers are arranged to set or adjust the threshold value(s) of the checking means inside the label and/or inside the label reader in accordance with that content code. The system according to the invention improves the Transfusion Safety (worldwide), and also for pharmaceutical and proofs objectively that the safe temperature requirements by law are 100% met. This means that not used pharmaceuticals can be re-used for a patient if not used by a particular patient.. This is a huge advantage over current procedures wherein now the pharmaceutical is destroyed). The invention also provides use of a label according to the invention or a method according to the invention or a system according to the invention or a container according to the invention to prevent a receptor from receiving blood, blood component or pharmaceuticals from a non-intended or wrong source and/or to collect heamovigilance data and information is the medication is taken by the patient. For recalling of medication or blood components, it is possible to determine the location of each individual blood- or medication.

The invention also provides a method to survey handling of a blood product comprising use of
a label for labeling a container suitable for containing said material, said label provided with information suitable for identifying said material, said label also provided with a monitoring device, and
a reader capable of providing a read-out of said monitoring device,
wherein
said monitoring device is provided with data suitable for identifying said blood product, and said monitoring device is capable of recording handling conditions that may be detrimental to the quality of said blood product, and
said reader is capable of providing a warning signal when said handling conditions have been detrimental to the quality of said product. In a preferred embodiment the invention provides a method wherein said monitoring device comprises an electronic article surveillance (EAS) device and said reader is an EAS reader.

Preferably wherein said monitoring device is a radio-frequency identification (RFID) device, preferably an active RFID device, and wherein said reader is an RFID reader, preferably said metod further comprising use of a writer capable of writing data in the monitoring device wherein said reader is also capable of storing said data from said read-out in said monitoring device and/or in said reader and/or in an external data storing device such as an hard disk or a system of hard disks, preferably wherein said storing device is a cloud-based device. It is much preferred that said monitoring device is capable of recording handling conditions temperature and/or handling time of said blood product. In a preferred embodiment of the invention said reader is capable of recording date of and/or time of and/or location of and/or identify a person taking part in providing said read-out. In a further embodiment said reader is capable of recording read-outs of at least two, preferably at least four, more preferably at least 10 monitoring devices that are simultaneously submitted to said reader. The invention also provides a label for use in a method according to the invention to survey handling of a blood product. Such a label, such as for labeling a container such as a blood bag preferably is provided with label information suitable for identifying a biological component and a monitoring device, such as an electronic article surveillance (EAS) device. In a much preferred embodiment, said label is provided with means through which tampering with said label is made evident. It is preferred that aid label information comprises information encoded with a barcode capable of identifying said blood product, for example as being plasma, platelets or red blood cells. It is preferred that said EAS device is a radio-frequency identification (RFID) device, and much preferred that said EAS device is provided with an antenna and/or power source. In a much preferred embodiment, the invention provides a label wherein tampering with said label results abolishing the function of said EAS device. In a preferred embodiment the invention provides a label according to the invention wherein said tampering results in disrupting a connection with antenna or power source of said device. The invention further provides a label according to the invention wherein said monitoring device allows for temperature sensing of the content of said container with the blood product and/or wherein said monitoring device allows for tracking and tracing said container. The invention also provides method to control quality of a blood product, a cell or cells, a tissue or an organ, comprising using a label according to the invention to label the container in which said blood product is stored or transported. The invention also provides a method to track and trace a blood product comprising using a label according to the invention to label the container in which said blood product is stored or transported. The invention also provides a method for providing a chain of documentation arising from the chain of events from the donation of donor blood to the transfusion of a blood product to a recipient comprising labeling a container comprising blood or a blood component with a label according to the invention. In a preferred embodiment, said method according to the invention comprises using at least a second integrated label after a processing step in the processing of said blood product, such as wherein said processing step comprises centrifugation or splitting of a blood product. The invention also provides a system to control quality of a blood product comprising using a label according the invention to label the container in which said product is stored or transported. The invention also provides a system to track and trace a blood product, said system using a label according to the invention to label the container in which said blood product is stored or transported. In a preferred embodiment, the invention provides a system according to the invention comprising using at least a second label after a processing step in the processing of said blood product and reading data from the log file of a first integrated label and importing said date into the log file of said second integrated label, for example when said processing step comprises centrifugation or splitting of a blood product. The invention also provides a container such as a blood bag provided with a label according to the invention, and provides use of a label according to the invention or a method according to the invention or a system according to the invention or a container according to the invention to prevent a receptor from receiving blood or blood component from a non-intended or wrong source and/or to collect heamovigilance data.

The uninterrupted cold chain, resulting from implementing the system according to the invention, of these living cells or plasma is a quality guarantee for the patient who is receiving a good blood component full of oxygen helping the patient to recover versus an unsafe blood or pharmaceutical component who can damage the patient. With the quality management system according to the invention returned and unused blood components (10-20%) left over after a surgery could be safely reused, so donor blood doesn't have to be wasted. If a blood component had been too warm or cold, causing an alarm code, there is management information to see where the cold chain could be improved, to prevent that the next time. The invention also provides a system according to the invention, wherein the label external control/reading means are arranged for transferring the blood temperatures and/or alarm codes read from the log file to label external processing means for further processing, logging, analyzing, displaying etc., preferably wherein the checking means are located in the label external control/reading means and/or the processing means. It is preferred that the blood bags are fit for the transportation of blood, blood plasma, blood platelets or red blood cells etc., wherein the integrated label also comprises means for storing a content code, such as a barcode, further identifying the blood bag's content or intended content.

The invention also provides a method to survey handling of a pharmaceutical product or pharmaceutical composition comprising use of
a label for labeling a container suitable for containing said product or composition, said label provided with information suitable for identifying said product or composition, said label also provided with a monitoring device, and
a reader capable of providing a read-out of said monitoring device,
wherein
said monitoring device is provided with data suitable for identifying said biological product or composition, and
said monitoring device is capable of recording handling conditions that may be detrimental to the quality of said product or composition, and
said reader is capable of providing a warning signal when said handling conditions have been detrimental to the quality of said product or composition. In a preferred embodiment the invention provides a method wherein said monitoring device comprises an electronic article surveillance (EAS) device and said reader is an EAS reader.

Preferably wherein said monitoring device is a radio-frequency identification (RFID) device, preferably an active RFID device, and wherein said reader is an RFID reader, preferably said metod further comprising use of a writer capable of writing data in the monitoring device wherein said reader is also capable of storing said data from said read-out in said monitoring device and/or in said reader and/or in an external data storing device such as an hard disk or a system of hard disks, preferably wherein said storing device is a cloud-based device. It is much preferred that said monitoring device is capable of recording handling conditions temperature and/or handling time of said pharmaceutical product or pharmaceutical composition. In a preferred embodiment of the invention said reader is capable of recording date of and/or time of and/or location of and/or identify a person taking part in providing said read-out. In a further embodiment said reader is capable of recording read-outs of at least two, preferably at least four, more preferably at least 10 monitoring devices that are simultaneously submitted to said reader. The invention also provides a label for use in a method according to the invention to survey handling of a pharmaceutical product or pharmaceutical composition. Such a label, such as for labeling a container such as a pill box or syringe or pharmaceutical product vial preferably is provided with label information suitable for identifying a biological component and a monitoring device, such as an electronic article surveillance (EAS) device. In a much preferred embodiment, said label is provided with means through which tampering with said label is made evident. It is preferred that said label information comprises information encoded with a barcode capable of identifying said pharmaceutical product or pharmaceutical composition. It is preferred that said EAS device is a radio-frequency identification (RFID) device, and much preferred that said EAS device is provided with an antenna and/or power source. In a much preferred embodiment, the invention provides a label wherein tampering with said label results abolishing the function of said EAS device. In a preferred embodiment the invention provides a label according to the invention wherein said tampering results in disrupting a connection with antenna or power source of said device. The invention further provides a label according to the invention wherein said monitoring device allows for temperature sensing of the content of said container with the pharmaceutical product or pharmaceutical composition and/or wherein said monitoring device allows for tracking and tracing said container. The invention also provides method to control quality of a pharmaceutical product or pharmaceutical composition, a cell or cells, a tissue or an organ, comprising using a label according to the invention to label the container in which said pharmaceutical product or pharmaceutical composition is stored or transported. The invention also provides a method to track and trace a pharmaceutical product or pharmaceutical composition comprising using a label according to the invention to label the container in which said pharmaceutical product or pharmaceutical composition is stored or transported. The invention also provides a method for providing a chain of documentation arising from the chain of events from the selection of the pharmaceutical product or pharmaceutical composition from the pharmacy to administering the pharmaceutical product or pharmaceutical composition to a recipient comprising labeling a container comprising the pharmaceutical product or pharmaceutical composition with a label according to the invention. In a preferred embodiment, said method according to the invention comprises using at least a second integrated label after a processing step in the processing of said pharmaceutical product or pharmaceutical composition, such as wherein said processing step comprises splitting of a pharmaceutical product or pharmaceutical composition. The invention also provides a system to control quality of a pharmaceutical product or pharmaceutical composition comprising using a label according the invention to label the container in which said product is stored or transported. The invention also provides a system to track and trace a pharmaceutical product or pharmaceutical composition, said system using a label according to the invention to label the container in which said pharmaceutical product or pharmaceutical composition is stored or transported. In a preferred embodiment, the invention provides a system according to the invention comprising using at least a second label after a processing step in the processing of said pharmaceutical product or pharmaceutical composition and reading data from the log file of a first integrated label and importing said date into the log file of said second integrated label, for example when said processing step comprises splitting of a pharmaceutical product or pharmaceutical composition. The invention also provides a container such as a pill box or vial or syringe provided with a label according to the invention, and provides use of a label according to the invention or a method according to the invention or a system according to the invention or a container according to the invention to prevent a receptor from receiving a pharmaceutical product or pharmaceutical composition from a non-intended or wrong source.

### Figure legends

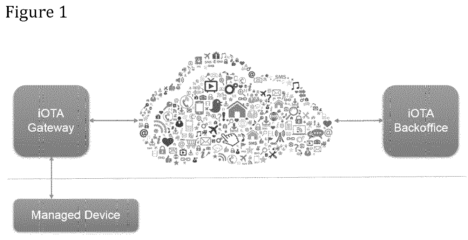

Figure 1. Global overview of the iOTA solution. The ICT iOTA solution is an end-to-end solution that comprises an iOTA Gateway and an iOTA Back office. The iOTA solution is built for scalability and high availability. Data and connections are all secured. The iOTA Gateway is a combined hardware and software solution that connects appliances, sensors and embedded devices to a central application platform.

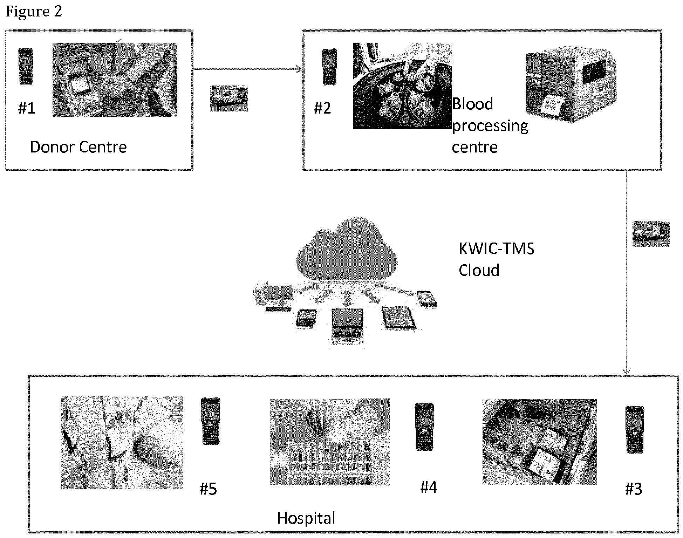

Figure 2. Global overview of Solution Description
Figure 3. Typical barcode and other information printed on an integrated label.
Figure 4. Overview of the chain of events from donor to receptor

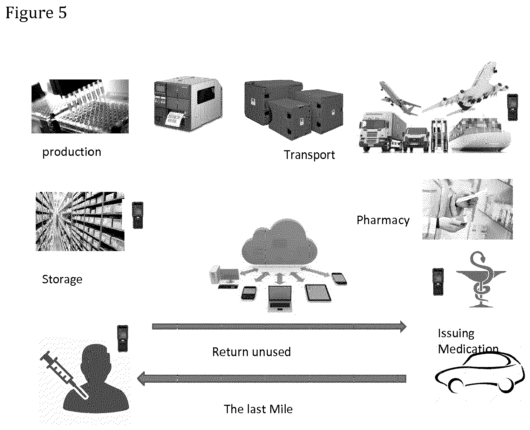

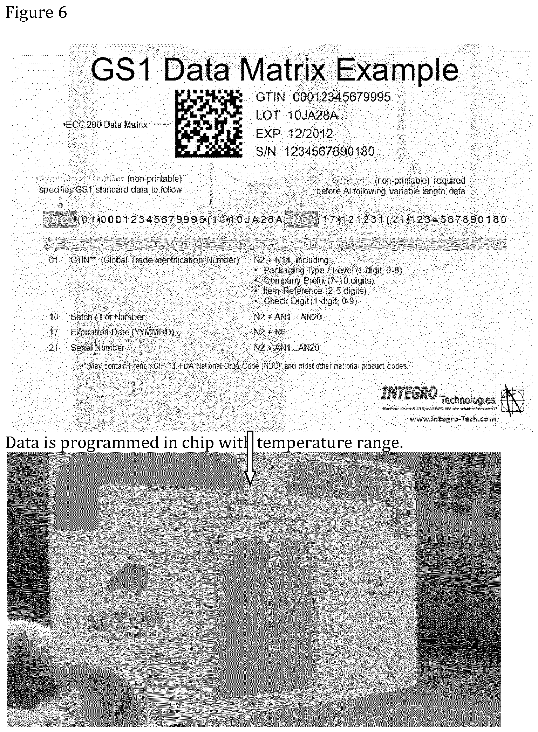

Figure 5 Currently, after production, medicine is sent to warehouse, with temperature on box level. Often there is no scanning on box level, and the trajectory from Pharmacy to patient, "The last mile", is not covered. Also, unused Medication, i.e. drugs that are not used in Pharmacy or at patient are destroyed because it is not known if temperature limits were exceeded! Furthermore, it cannot be ascertained if patient takes medication. A situation that is complicated by the fact that generally, from all the patients admitted in a hospital, only 7% has a correct medication file.
Figure ., KWIC_TMS solution, every medicine with standardized label with UID:

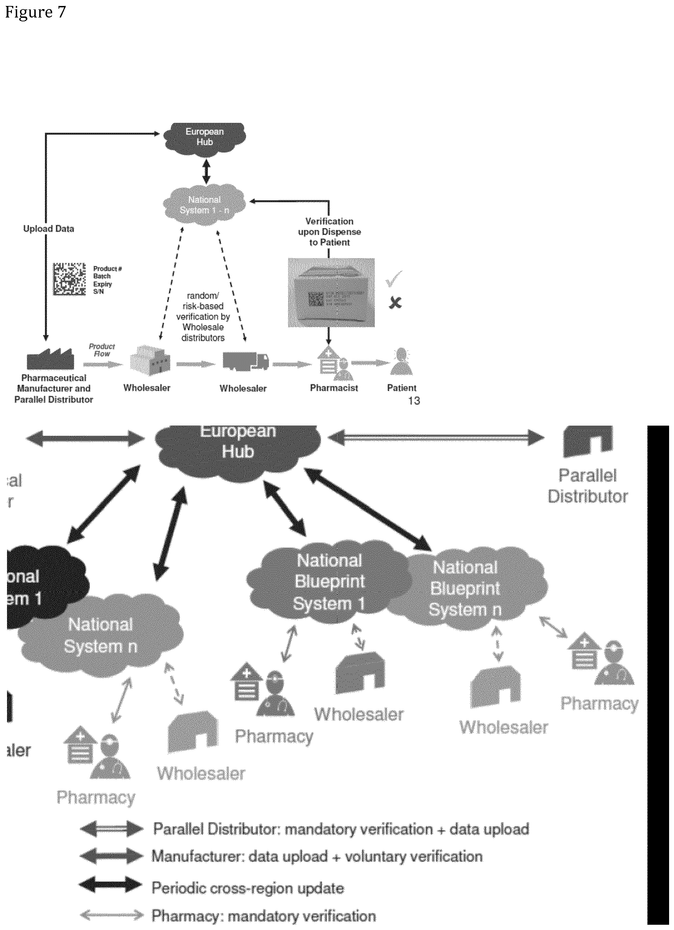

Figure 7. Overview of routing of medicine

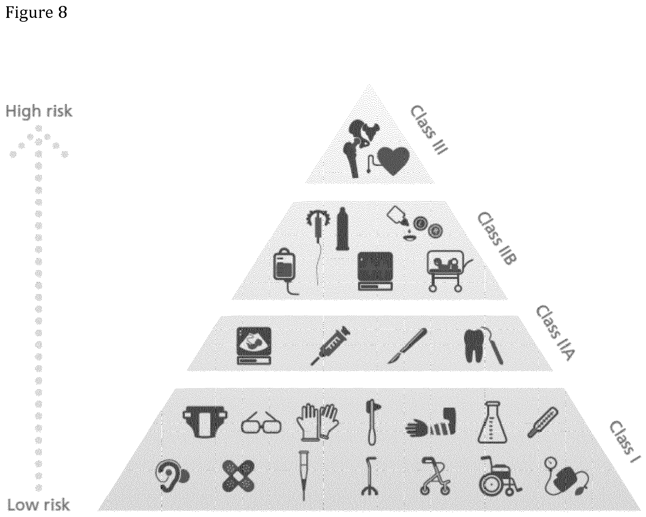

Figure 8. Risk classifications of medicinal supplies

### Detailed description

### Transfusion procedures

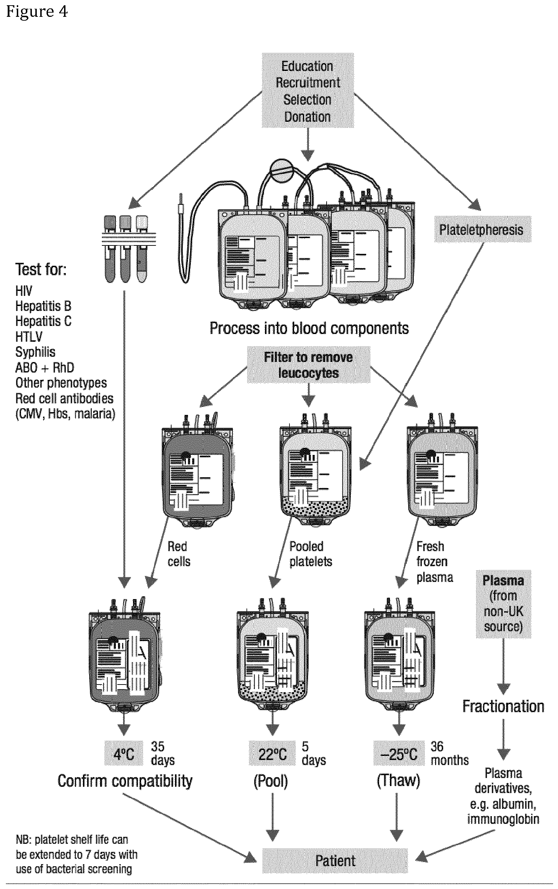

Transfusion of blood components is a complex process involving multiple departments, staff members, and steps. Accurate recipient identification at blood collection and administration is essential to the safety of the total blood transfusion process (see also figure 4). Facilities may consider risk reduction strategies that address inappropriate prescribing prctices, correct patient identification of the transfusion candidate, and clear lines of responsibility. Whole blood is a term used in transfusion medicine for human blood from a standard blood donation. The blood is typically combined with an anticoagulant during the collection process, but is generally otherwise unprocessed. In the US, the capitalized "Whole Blood" means a specific standardized product for transfusion or further processing, where "whole blood" is any unmodified collected blood.

Historically, blood was transfused as whole blood without further processing. Most blood banks now split the whole blood into two or more components, typically red blood cells and a plasma component such as fresh frozen plasma. Platelets for transfusion can also be prepared from a unit of whole blood. Some blood banks have replaced this with platelets collected by platelet pheresis because whole blood platelets, sometimes called "random" platelets, must be pooled from multiple donors to get enough for a therapeutic dose. Platelet pheresis is the process of removing whole blood from a donor, separating the blood into its components, keeping the platelets, and then returning the remaining blood components to the donor.

The collected blood is generally separated into components by one of three methods. A centrifuge can be used in a "hard spin" which separates whole blood into plasma and red cells or for a "soft spin" which separates it into plasma, buffy coat (used to make platelets), and red blood cells. The third method is sedimentation: the blood simply sits overnight and the red cells and plasma are separated by gravitational interactions.

### Clinical background of transfusion disease.

Transfusion-related acute lung injury (TRALI) and transfusion-associated circulatory overload (TACO), are the two leading causes of blood transfusion-related deaths in the United States. Researchers report that postoperative TRALI is significantly underreported and more common than previously thought, with an overall rate of 1.4 percent. While the rate of TACO may be on the decline, there is still significant risk to surgical patients. In 2011, nearly 30 million blood components were transfused in the US, half of which occurred in the operating room. TRALI and TACO mostly occur after intra operative blood transfusions. TRALI occurred in approximately 1.4 percent of surgical patients. Incidence rates were higher in those having surgery inside the chest cavity, on major blood vessels, or having an organ transplant. Surgical patients who received larger volumes of blood were also found to be at increased risk. TACO occurred in 4.3 percent of surgical patients, with higher rates associated with increased volume of blood transfused, advanced age and total intra operative fluid balance. Again, patients having surgery inside the chest cavity, on major blood vessels, or organ transplants were at greatest risk. The FDA reports that approximately 38 percent and 24 percent of deaths after transfusion in 2012 were because of TRALI and TACO, respectively.

### Shelf live and temperature thresholds of blood and blood components

Security temperature thresholds (limits) and maximal shelf life values may have different values for the various blood products, viz. (on average):

| *Content* | *Content code* | *Security thresholds* |
|---|---|---|
| Blood | 1 | max. 18 - 24C°; max. 3 days |
| Blood plasma | 2 | -50C° to -25C°; max. 2 years |
| Blood platelets | 3 | 18C° to 25C°; max. 5 days |
| Red blood cells | 4 | 2C° to 10C°; max. 40 days |

### Integrated label with RFID inlay.

The RFID label in an integrated label comprises the chip and aluminum, copper or silver antenna bonded to a polyethylene terephthalate (PET) layer that is delivered to the label maker "dry" (without adhesive) or "wet" (attached to a pressure sensitive liner). The label preferably comprises an RFID (EPCGlobal C1G2 and ISO18000-6C standard) active RFID chip and a battery. The label can be printed with information in text or barcodes (1D or 2D) For an example of printed information see Figure 3. The RFID label preferably has a memory for data and a memory for logging temperature data, a temperature sensor, algorithms to calculate temperatures, and programmable alarm levels for high and low temperatures. The RFID label can be programmed during printing (RFID printer) with data and alarm levels or separately with a handheld RFID device.

### Tampering

If the label is removed from the asset where it is placed on, the label will be destroyed because the antenna, chip or battery will be torn into pieces because of the glue that attaches the different items of the integrated label.

### Open standard

The integrated label is preferably open EPC and/or US standard and can be used for tracking and tracing and temperature control of biological products (such as a blood product).

### Additional information

The integrated label can contain information about the product type, origin and maximum and minimum temperature ranges. The temperature during activation is stored in the label.

The integrated label makes use of a temperature monitoring system that can program the label, read the label and determine the route of the asset were the label is attached at, the temperatures, the locations.

The locations are determined by reading the label; the readers have defined locations that are stored in the temperature monitoring system cloud application.

The temperature monitor system keeps track of the position (routes, trips), temperature, expiration date etc. during these trips and alarms persons that are defined in the temperature monitor system who are responsible for this process.

The temperature monitoring system can generate detailed reports about the trips. Both shorter trips of each product liable institution in the Transfusion Chain, but also the overarching life cycle of the blood bag (40 days) of the transfusion chain.

The integrated label can be used for transfusion administration by checking blood type of donor (origin) and expiration date and allowed temperature and time over and under temperature in relation with the blood type of the patient receiving the blood by barcode or RFID identification on wristband of the patient as a bedside quality check before transfusion takes place, to prevent that the patient receives too warm blood that causes injury. Various useful RFID tags, reader, antenna and/or writer configurations are identified herein below.

The integrated label consists of software that gives the status of the process:
Dormant, waiting for start
Active, logging in process
Logging trip Stopped
Trip start
Hardware failure
Battery low
Logging stopped battery exhausted
Status ok, low temperature, high temperature exceeded, time start exceeding

Activation commands:
Start trip
Start new section in trip
Stop section in trip
Stop section and complete trip
Read trip
Read component data (donor, origin, expiration date, component id)
Write low temperature limit
Write high temperature limit
Write component data (donor, origin, expiration date, component id)
Check temperature and give status
Read temperature data, all, between low/high, between data/time.

During the trip, temperature can be checked by a hand reader such as a personal digital assistant (pda) with RFID and barcode reader that has unique number that is stored in system together with location and staff-ID so that data can be read and stored and the location and temperature can be tracked and traced on different locations during the complete trip.

This way also the part of the trip where the temperature was exceeded is determined so the cause can be solved by analyzing data (time, temperature, location, responsible staff).

If a problem occurs, the responsible manager receives a message and he can analyze the data, location and temperature, so he can improve the process.

The data can also be used for financial and management reports.

### Process description:

### Store material in container or bag

Print label with data, barcodes and program data in RFID integrated label with RFID printer or handheld RFID programmer that can also contain a barcode reader to identify the bag or container.

During the trip, at every point were the bag or container is sent or received, the data in the integrated label is read by a (optionally handheld) reader or device that can also comprise a barcode reader to identify the staff member handling the bag or container.

If the temperature is or has been exceeded (or below the minimum value), a warning is given to the user on the handheld device so he knows the material cannot go further in the logistic process. A message is send to the responsible manager.

If the material like full blood is split into different components during the process, the original Identification is stored in the new label that is produced after splitting and the new data like product number, location, expiration date etc. is also programmed in the RFID chip by RFID printer or handheld device.

If the material or product is admitted to a patient, the data is also read by a (handheld) reader to check is temperature or expiration date is not exceeded or the temperature was to low according to the data stored in the RFID chip. If the limits are exceeded or the expiration date is exceeded, a warning is given to the staff member so that he knows the product cannot be admitted to the patient. The responsible manager also gets a warning so that he can analyze the problem.

The system can also be used for admitting identity of the patient by checking patient id that can be stored in another system and were the matching is checked and stored between patient and product or material.

### Specifications and global overview.

This section describes the TMS solution in terms of its external visible building blocks. The solution is designed to be modular. Building blocks can be replaced while maintaining the overall solution. The global overview shows the following components: Blood component with temperature monitoring device. The temperature-monitoring device is a RFID chip with temperature sensors. It can, when configured and activated, autonomously sample the environmental temperature in a given time frame. For TMS the RT0005 (supplier CAENRFID) is for example selected. The chip has on-board memory for storing temperature samples and other configuration settings. It has an LED to indicate whether certain thresholds are expired.

Another example is the Confidex temperature Monitoring label® that has about the same specifications but has the shape of a credit card, but without a LED.

Decisions on accepting or rejecting blood components can be done without the other components of TMS. For communication with external components it uses the UHF RFID standard.

### RFID Reader

The RFID Reader is a (handheld) device that can be used by (medical) personnel to configure and activate the temperature monitoring device, configure the device with the temperature ranges that are safe, critical and un-safe, the sample rate, activate the temperature monitoring functionality of the device, and so on.

### Scan the temperature-monitoring device.

All sample data, including temperature values and time stamps are read from the device. The RFID Reader can also indicate whether the blood component is to be rejected or not.

### Upload sample data to the central data store

When an Internet connection is available, (for example when the reader is equipped with Wi-Fi), available sample data is sent over a secure connection to the central system.

For TMS in this example the DOT300C is selected. The DOT300C is a touch screen device that has two scanning options: UHF RFID and one-dimensional bar codes. The operating system is Windows Mobile 6.5.

### Back office

The back office is the central component that allows for the management of TMS. It has a number of tasks:
Collecting and storing temperature monitoring results
Managing configurations for the temperature monitoring devices
Managing authorization and authentication settings for TMS users.
Presenting TMS data to users.
The back office is located in the cloud. All data is centrally stored.

### Client Devices

The back office offers web-based access to users. Through client systems with browsers the users can, depending on their roles,

### Inspect and manage all data of the back office (system users on PC based systems)

Inspect and manage only data of the back office that is owned by the organization to which the user belongs. (End-users on PC based systems and on mobile tablets) The data is presented in two ways
In data grids that allow for sorting, filtering, editing, creation and CSV exports
In time-temperature graphs (per temperature device per trip).

### RFID reader Device Management System

Temperature monitoring device - RFID Reader

This interface has two components:
Radio interface UHF RFID EPC C1G2 (ISO18000-6C)

This interface allows for reading and writing memory locations and registers on an RFID chip. The semantics of the locations and registers are manufacturer specific.

### Optical barcode reading

This interface allows for optical (one-dimensional) barcode reading.

### RFID Reader - back office

This interface is a REST-Api web service interface, HTTPS based. The back-office presents an URL (e.g. https://backoffice.kwic.nl). The interface is security-protected and encrypted, the security-mechanism is to be decided (username/password or client certificates)

Back office - Client device. This is an HTTPS based HTML interface (standard browser technology), username/password protected.

New RFID tracking and monitor system process from donor to patient, further specifications.

The system preferably consists of a system (KWIC TMS Tracking and Monitor System) that is housed in the cloud.

The system preferably consists of an RFID reader/Writer from which the distribution location in the transfusion chain is programmed in the system (in NL 11 times in and out the cooling systems before the bedside check at the transfusion patient). The reader contains a program that can read/write multiple RFID labels with real time temperature measurements.

The reader can communicate with the cloud system by a wireless connection, if there is temporary no connection, the data is stored in the reader until the connection is established. The reader can check stand alone the temperature and expiration data because the program that handles that is stored locally on the reader. So it can be used stand- alone.

The system consist of many RFID tags that can contain temperature triggers or loggers and necessary data like expiration date, donor id and component type number.

#1: when the donor is coming to the donor center (or mobile donation center), he/she receives a sheet with barcode stickers with a unique donation number and donation centre id. This donation number is registered with the donor name and birthday in a system.

There are a couple of tubes with blood taken from the donor to check for a couple of deceases.

The label with donation barcode number is taken from the sheet and stickered on the tube for identification and on a donation form.

=> A new RFID label is stickered on the blood bag, the donation number and blood center number is scanned with a RFID reader/writer and the RFID chip and is programmed with the donation number, and the blood component type while it is printed out. The RFID chip starts logging the temperature for full blood (18-24 C). The data is stored in the KWIC system in the cloud that is connected wireless by Wi-Fi or gprs or similar

If there is no wireless connection the data is stored locally on the RFID reader and send when the connection is established.

If the blood is transported to a blood processing center, the bags are put in a box (#1,#2). The box with all the bags is scanned with an RFID reader in 1 read process (multiple reads complete box with all the bags). The bags ID and donor ID are written in a system, the temperature of the bags is checked at the same time. If 1 or more bags exceeded temperature, an alarm is given on the RFID reader and the bag id and temperature is displayed on the display of the reader the data is stored in the KWIC system in the cloud were is connected by wireless (Wi-Fi, gprs or similar). Also the location of the reader is stored in the system with the reader ID, so the location of the blood supply is known.

The temperature is determined by the reader based on the component type number from the RFID chip, this determines the temperature range for each blood component.

The reader can function stand alone for checking without a wireless connection because the program is stored in the reader,

If there is no wireless connection the data is stored locally on the rfid reader and send when the connection is established.

This barcode is then read by a robot that takes samples for testing in the lab.

For full blood donation, the blood is taken from the patient in the blood bag that is also connected with 3 satellite bags (1 for platelets, 1 for plasma and 1 for red blood cells).

On each of these blood bag is a worldwide standard production label that the producer of the blood bags has put on the bags during production with lot number, producer, donation centre etc.

A sticker with the donation number from the donor is put on all 4 blood bags.

For plasma donation the donor is connected to a machine that filters the plasma from the blood and returns the filtered blood back to the patient. Only 1 bag is used. This plasma has to be frozen as soon as possible at minus 50C, before it is frozen, the barcode is scanned and the bag is put in the fridge.

=> for plasma A new RFID label is stickered on the blood bag, the donation number and blood center number is scanned and the RFID chip is programmed with the donation number. The RFID chip starts logging the temperature for unfrozen plasma (18-24 C). When the plasma is put in the fridge, the bag or bags are scanned so that the time and temperature from unfrozen until frozen is known because this is a critical temperature for plasma., this data is send to the KWIC system. The frozen rfid chip stops at a certain low temperature, but the data is saved. If the temperature rises, the rfid chip starts working again and can be read when it is taken from the fridge and goes on transport.

After the donation is checked for deceases and the full blood is ok, the 4 blood bags are put in a centrifuge to split the components in the donation bag #2.

Then the blood bags are connected to a splitting machine were the different components are separated in the 3 satellite bags.

A new RFID donor label is printed with the donation number, the component code and the expiration date of that blood component. At the same time the label is printed, the data is programmed in the chip (RFID printer). This label is put on each blood bag.

=> now is decided for plasma A new RFID label is stickered on the blood bag, the donation number and blood center number is scanned and the RFID chip is programmed with the donation number. The RFID chip starts logging the temperature for unfrozen plasma (18-24 C).

For platelets, the platelets of 5 donors is mixed and a new RFID label is generated that identifies the 5 donors.

The batch of bags can be scanned in 1 multiple read before they are stored, the location is also send to the KWIC system based on the location of the reader-id.

The blood is stored in a temperature-controlled environment; the platelets are shaken on a machine to keep the condition. If the blood is stored, the RFID data is scanned and the location is stored in a system.

If a hospital orders blood, the blood bags that are selected, the RFID tag are scanned and the data is stored in a system. The blood bags are put in a box and transported to the hospital. The time, date location temperature etc. are send to the KWIC system.

#3 when the ordered blood is received by the hospital. The box is scanned by a reader in 1 multiple read while still in the box or on the table. The temperature is checked at the same time, the location, date, time, component code, donor id etc. are send to the KWIC system and can be stored in the LIMS.

If there is one or more bags who exceeded the safe temperature, the data is displayed on a reader and the data is send to the KWIC system. If there is no connection the data is send when the connection is established.

Then the bags are put in a temperature controlled fridge. The bags are scanned with location and data from the bags are send to the KWIC system. Now is known where which bag is.

When blood is ordered from the lab (or blood bank), blood bags with the type of component that is needed is taken from the fridge the rfid DATA is checked (also temperature and expiration date) and the blood is cross checked with the patients that needs the blood. Also an AB0 screening is done often.

Blood is taken from the tubes that are connected to the blood bag and are put is a glass tube for analysis. #4

After this check the donation number is connected with the patient number and in the LIM system.

The patient ID can be programmed in the RFID chip with a reader but is not necessary because at the bedside the donor number can be checked against to donor/patient data in the lims system.

The RFID of the bag is scanned and the temperature is checked, the data like location etc is send to the kwic system. This bag is put back in the fridge until it is taken to the ward with the patient.

If the blood is taken from the fridge to transfuse it to the patient, the RFID bag is scanned and the data is send to the KWIC system, the expiration date and temperature is checked.

The blood component is taken to the ward and must be admitted within 30 minutes; else the blood has to be destroyed.

At the bedside #5, the nurse barcode badge is scanned, the patient wristband is scanned and the blood bag RFID label is read:
The expiration date, and temperature and donor number are checked is a multiple read (barcode and RFID) this is send to the KWIC system. The reader can also be used for present lim systems were interfacing we be possible with the KWIC system to check the data.

This data can be sent to the LIMS where patient and donor ID are checked.

A patient id label is scanned. If the patient id on the blood bag matches the patient id of the wristband, the transfusion is started. The data is stored in the LIMS system. The temperature of the patient, the blood pressure and the heart rate is checked and logged in a form or system. This is also repeated after 10 minutes after transfusion start.

If the blood is not admitted to the patient, the blood is returned to the laboratory or blood bank were the RFID chip is read and the temperature and expiration date are checked. If the temperature and date are ok the blood bag is put bag in the fridge so it can be reused by other patients.

If there is a recall from a blood bag from a certain donor, the blood processing centre can see in the KWIC system exactly where the blood bag is in the logistic chain, but if it is in a patient this is also know by the patient ID.

The blood-processing center can see exactly where all the blood bags are and also knows exactly were which type of blood is in the logistic chain.

The Hospital management can get data (heamovigilance) about all the donations on every ward and how much blood is used, destroyed or re-used.

With this system the blood processing centers and hospital comply to GMP and the EU-law 2004/23/EG from the European Parliament and also product liability of the produces (blood processing center) (currently that is not the case).

### Further advantages

With our temperature monitoring system, logistic process, software and RFID technology, we reduce structural donor blood waste. We also make the whole transfusion process from donor to patient safer than ever, setting a new worldwide standard. Our quality management system is a new market application suitable for blood, cell, tissue, organ products, medicines and raw materials.

Blood can be safely reused with this kind of quality management when left over after surgery (15% worldwide) if the safe temperature profile and safe expiration date is monitored. This is saving money and precious blood and does justice to the donor. (In the EU are about 15 million transfusions on a yearly base)

We improve the transfusion safety severely by protecting the entire transfusion chain (overarching product liable institutions) from donor to transfusion patient, where the patient will for the first time ever, have a bedside quality check before the transfusion takes place. This is preventing injury. The system integrates the old barcode and new RFID techniques into the standard worldwide blood bag label (ISBT 128 protocol), providing temperature control, expiration date, tracking and tracing, patient identification (right product, right patient) and blood component check.

By using open standards (EPC Gen II), every producer of blood transfusion systems can integrate this new label in their system or use the complete KWIC-TMS transfusion safety system. This will make an end to all different transfusion safety systems with their own standards, so this open standard will be quickly widely accepted and is easy to use. It provides:
From Donor to Blood Bank: Traceability data, Haemovigilance and Temperature Control
From Blood Bank to Hospital: Traceability data, Haemovigilance and Temperature Control
From Donor to Transfusion Patient: Patient Identification, Blood Component Check, Expiration date of the Blood Component, Temperature History, Haemovigilance and Traceability data.

From bedside to intake leftover blood to reuse: Expiration Patient Identification, Blood Component Check, Expiration date of the Blood Component, Temperature History, Haemovigilance and Traceability data.

To prevent the unnecessary waste of costly human resources by safely reusing returned blood components. Enabling more sustainability of human blood and lower costs of healthcare. (also suitable for samples in Bio Bancs).

To improve patient safety structurally, by preventing that the transfusion patient receives too warm Red Blood Cells that are bacterial contaminated causing injury like Sepsis and causing damage to human organs.

To prevent death and complications by sepsis and the high healthcare cost for extra Hospital days (€300, - per day in NL) and at the intensive care unit (€2000,- a day in NL) because of the complications caused by too warm blood resulting in post transfusion sepsis and organ damage. Blood of bad quality also degrades the human immune system.

To prevent that the patient receives the wrong blood product: Until now >4 of every 1000 transfusion patients die because receiving blood from the wrong donor.

To apply and control the existing European regulations (2002/98/EG, 2001/83/EG) for storing, transporting and handling blood components. That ensures save temperature limits for blood components in all European countries.

Requirements for storage and transport for blood components:
Full Blood: maximum of 3 days between 18 and 24 degrees Celsius.
Plasma: maximum of minus 25 degrees Celsius for 2 years
Platelets: between 18 en 24 degrees Celsius for a maximum of three days
Red Cells: between 2 en 10 degrees Celsius for a maximum of 40 days

For example: the Red Cells that are used in hospitals must be kept in a winter sleep between 2 and 10 degrees Celsius. Higher temperature causes the increase of bacteria and deterioration of blood cell quality causing damage to the patients' organs and can lead to post transfusion sepsis. In the Netherlands, 3700 people a year are dying by sepsis.

The following references are hereby incorporated by reference in their entirety.
US patent application 11/795,677
US patent US 7095324
US Patent 05850181
3M product specification for 7900 Destructible White Vinyl Film Sheet.
3M product specification for 7930 Destructible Vinyl Label Material.
3M product specification for 7610 ScotchMark Destructible White Vinyl.
3M product specification for 7847 Laser Markable Tamper Evident Material.
3M product specification for 7385 ScotchMark Tamper-Indicating Dot-Matrix Impact Printable.
3M product specification for 7866 Tamper-Indicating Void Polyester.

### Further detailed product specifications.

New label KWIC-TMS2 label (tag) based on Iceberg trace-tech Label and developments to KWIC-TMS 1 and 2 labels. Present label has trigger, new has same functionality as kwic-tms1 label and additional features like data logger and shelf life.

*KWIC-TMS1 LABEL* is capable of monitoring temperature in real time.

When Maximum and Minimum temperature ranges are configured, an alarm flag will be set in one word of the Users memory if the temperature ranges are crossed.

It is easy to target if a product has not followed the temperature conditions we are expecting.

### Capabilities of temperature BAPS (battery assisted) :

- An integrated temperature sensor included on the chip supports the temperature range on -40ºC to + 64ºC.
- Many tags can be checked at the same time.
- Bap tag life can be adapted to every costumers need adjusting price and performance.
- BAPs have better reading performance than passive tags.
- Most of the battery's energy is consumed only when the BAP is under the RFID field, monitoring temperature is cheap in terms of energy consumption.
- Monitoring and BAP energy consumption can be turned ON and OFF
- BAP temperature has an integrated UTC clock, if a flag is set we can find out when it happened.
- BAPs can be reset and temperature limits changed as many times as we like ass long as the battery is alive.
- The chip in the BAP follows EPC global Gen2 standard

### Specifications:

ISO 18000-6C (Gen2) & 18000-6D (TOTAL) compliant
- EPCTM Gen2 compliant
- AIAGTM B-11 compliant
- ATA Spec 2000 Low Memory Tag compliant
- 4096-bit non-volatile memory (EEPROM)
- 48-bit manufacturer programmed IC Serial Number
- 352 bits for UII/EPC encoding
- 3072 bits for User data / 3008 bits for TOTAL data
- 128-bit Register File
- BlockPermalock command for User memory
- Forward link data rates: 26.7 to 128 kbps assuming equiprobable data
- BlockErase and BlockWrite commands for high speed memory transactions
- Return link data rates: 40 to 640 kbps with subcarrier modulated data rates of 0.625 to 320 kbps
- Battery assistance for superior reading range and reading reliability
- Rectifier that allows purely passive operation in case the battery is flat or not present
- Battery supply management to prolong battery life
- Battery supply range: 1.25V to 3.6V
- Low battery alarm threshold: 1.3V or 2.2V
- 32-bit password-protected Kill command
- 32-bit password-protected Access command
- Extended temperature range: -40 °C to +85 ° C

New label KWIC-TMS2 label based on penguin from trace-
tech-id with data logger
Benefits
Versatile temperature and data logging
Worldwide EPC compliant
Works fully passive or in BAP mode
Programmable logging modes with various sensors
Works with EPC readers EPC
Provides supply for external sensors
Autonomous data logging with timestamp
Sensor alert function External
Supports fast communication via SPI
Storage for up to 841 events with timestamps
Alert for shelf life expiration
Programmable sensor limits
Features
High Temperature Range: -40°C to +125°C
Frequency: 860 to 960 MHz
Battery supply: 1.5V
Data logging from On-chip temperature sensor
and 2 external sensors
Class 1 and Class 3 Compliant
Energy harvesting from reader field
Real-time clock for data logging
sensor interrupt capability
Serial peripheral interface
On-chip 9k bit EEPROM
Integrated dynamic shelf life calculation
Advanced logging with 4 user-selectable limits
EM4325 IC
18000-6 Type C (Gen2) and Type C/D (Gen2/TOTAL) RFID IC

Typical Applications
tag with temperature reading on demand.
tag with tamper detection and temperature reading on demand.
tag with EM4325 as SPI Master with energy harvesting to power another component as SPI Slave.
BAP tag with tamper detection, temperature monitoring, and alarm indicators.
BAP tag or embedded application with EM4325 as SPI Master and another component as SPI Slave
BAP tag or embedded application with EM4325 as SPI Slave and another component as SPI Master.

### Description

EM4325 is a Class-3 Generation-2 (Gen2) IC that is compliant with ISO/IEC 18000-6:2010 Type C and Type D (TOTAL) as well as EPCTM Class-1 Generation-2. The chip offers an advanced feature set leading to a performance beyond that of standard Gen2 chips and can be either battery powered or beam powered by the RF energy transmitted from a reader. In a battery assisted passive (BAP) configuration, the EM4325 offers superior reading range and reliability compared to purely passive RFID solutions.

EM4325 includes 4096 bits of high-speed non-volatile memory (EEPROM) that is organized into 64 pages with 4 words per page. The chip supports either ISO or EPCTM data structures that are compliant with EPCglobal Tag Data Standards, Version 1.6, and is delivered with a Unique Identifier (UID) to ensure full traceability.

An integrated temperature sensor is included in the EM4325 and supports the temperature range from -40 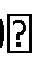 C to +60 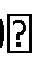 C. The temperature sensor may be used in either purely passive or BAP applications. A reader can make temperature readings on demand or the chip may be programmed to perform self-monitoring with alarm conditions. The temperature sensor may also be configured to provide Simple Sensor Data reporting using temperature sensor formats defined in ISO/IEC 18000-6:2010.

EM4325 supports advanced applications by providing programmable external interfaces for an auxiliary function and a 4-bit I/O port. The auxiliary function may be configured as an input for tamper detection or as an output for notification of RF events to external devices. The 4-bit I/O may be configured to support 4 discrete signals or as a Serial Peripheral Interface (SPI) bus. The chip may serve as either an SPI Master device or an SPI Slave device. The programmable external interfaces allow the EM4325 to function as an RF front end and protocol handler in advanced RFID tags or embedded applications. In a passive configuration, the programmable external interfaces allow the EM4325 to serve as a SPI Master with energy harvesting and provide power to external components.

Battery supply management is provided to prolong battery life in BAP applications. The chip supports programmable duty cycle control, auto-switching between battery powered and beam powered operation, and programmable enable/disable of an ultra-low power mode for extended storage applications.
EPC is a trademark of EPCglobal Inc.
AIAG is a trademark of Automotive Industry Action Group
Features
ISO 18000-6C (Gen2) & 18000-6D (TOTAL) compliant
EPCTM Gen2 compliant
AIAGTM B-11 compliant
DATA Spec 2000 Low Memory Tag compliant
4096-bit non-volatile memory (EEPROM)
48-bit manufacturer programmed IC Serial Number
352 bits for UII/EPC encoding
3072 bits for User data / 3008 bits for TOTAL data
128-bit Register File
BlockErase and BlockWrite commands for high speed memory transactions
BlockPermalock command for User memory
Forward link data rates: 26.7 to 128 kbps assuming equiprobable data
Return link data rates: 40 to 640 kbps with subcarrier modulated data rates of 0.625 to 320 kbps
TOTAL data rates: 64, 128, 160, 256, or 320 Kbps
Coordinated Universal Time Clock (UTC)
Integrated temperature sensor: -40C to +60C with typical accuracy of ±1.0C over the full range and ±0.6C over the ISO range for cold chain
Programmable monitoring and alarm conditions for temperature sensor including time stamp
Programmable auxiliary function: input for tamper detection or output for notification of RF events
Programmable 4-bit I/O port: configurable as 4 discrete signals or as a Serial Peripheral Interface (SPI) Bus
Battery assistance for superior reading range and reading reliability
Rectifier that allows purely passive operation in case the battery is flat or not present
Battery supply management to prolong battery life
Battery supply range: 1.25V to 3.6V
Low battery alarm threshold: 1.3V or 2.2V
32-bit password-protected Kill command
32-bit password-protected Access command
Extended temperature range: -40C to +85C

### Applications

### RFID tags:

Supply chain management, tracking and tracing,
reusable containers and pallets, access control,
asset control, cold chain monitoring, sensor monitoring,
E-seals, Gen2 side-channel for active RFID tags

### RFID front end for embedded applications:

Gen2 communications channel for wireless data
Exchange, configuration and control, RF event
notification

### CS771 Long range UHF RFID antenna

### Features:

Industry leading read range performance far field antenna
Circular polarization, choice of Left Hand Circular Polarized or Right Hand Circular Polarized.
Global frequency coverage
Excellent matching for best read performance

### Product Profile:

CS771 long range far field antenna is a
monostatic antenna for use with CS461 reader
for ultra long range high sensitivity tag
reading. The antenna has industry leading
circular polarization properties to enable best
read of tags of all orientations as they
approach the sweet zone of the antenna. The
antenna has excellent beam width and frontto-
back ratio.

### Specifications:

### Physical

### Characteristics:

Length = 30 cm; Width = 30 cm; Height = 4 cm; Weight = 5 lbs;
Mounting: 2 x 2 mounting stud, horizontal separation 5.875 inches, vertical
separation 2.75 inches, ¼ x 0.625 inch studs with 20 threads/inch
Read Range: With CS461-2 & AD431 tags from Avery Dennison, 8 meters outdoor
Frequency Range: One of the following: 865-868 MHz, 865-867 MHz, 902-928 MHz, 952-
954 MHz
Polarization: RHCP or LHCP
Axial Ratio: Less than 2 dB
Peak Linear Gain: 6 dBiL
Gain Flatness: 0.5 dB
Beamwidth (3 dB): 54 degrees
Input Impedance: 50 ohms
Input Power: 10 Watts
ESD: 10 KV (no ESD sensitive components inside)
Environment: Operating Temp: -200C to 550C (-40F to 1310F)
Storage Temp: -400C to 850C (-400F to 1850F)
Humidity: 10% to 95% Non-condensing
Order Code: CS771C-024-(L/R)HCP-N (N=1: 865-868 MHz for Europe & 865-867
MHz for India, N=2: 902-928 MHz, C=S:SMA Reverse Polarity
Connector, C=N:N Reverse Polarity Connector, C={none}: TNC
Reverse Polarity Connector, 024=standard 2.4m cable and please
discuss with CSL sales department for special requirements) 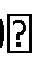
Excellent circular polarization characteristics for reading tags of all orientations

### Speedway R420 UHF RFID Reader

### ELECTRICAL:

### Air Interface Protocol:

EPCglobal UHF Class 1 Gen 2 (ISO 18000-6C)

### Operating Frequency:

UHF 860- 960 MHz, region dependent

### Transmit Power (POE):

+10.0 to +30.0 dBm

### Transmit Power (External DC Power):

+10.0 to +32.5 dBm

### Max Receive Sensitivity:

-82 dBm

### Max Read Distance:

N/A

### Max Read Rate:

N/A

### Data Interface:

RS-232, ethernet

### GPIO:

4 GPI optically isolated 3-30V/ 4 GPO optically isolated, 0-30V

### Power Source (POE):

IEEE 802.3af

### Power Source (External DC Power):

+24 VDC @ 800mA

### MECHANICAL:

### Antenna Ports:

4 RP-TNC, monostatic

### Dimensions:

7.5 x 6.9 x 1.2 in (190.5 x 175.3 x 30.5 mm)

### Weight:

1.5 lbs (680.3 g)

### ENVIRONMENT:

### IP Rating:

IP 52

### Operating Temperature:

-20 ºC to +50 ºC

### APPLICATION INTERFACE:

### Host API:

.NET

### FEATURES

Autopilot - a set of unique firmware features work together to automatically optimize the reader's operation for its environment.
Autoset - continuously optimizes the reader's configuration for the best, most reliable performance
Low duty cycle - reduces RF interference, power consumption, and energy costs Dynamic antenna switching - improves throughput and helps the reader work more efficiently
Power over Ethernet (PoE) - simplifies deployment and dramatically reduces cost by eliminating the need for AC outlet installation at read points.
Improves upon the advanced hardware capabilities that made the original Speedway the reader of choice for many demanding customers-capabilities such as best receive sensitivity, interference rejection, and item-level carrier cancellation.

### The RFID UHF A1001 Near Field Antenna

**Physical / Environmental Specifications**

| | |
|---|---|
| Dimensions (LxWxD): | 82.0 mm x 82.0 mm x 9.5 mm |
| | 3.2 in. x 3.2 in. x 0.3 in. |
| Weight: | 100 g / 0.22 lbs. |
| Casing: | Moulded ABS housing |
| Environmental Rating: | IP65 |
| Operating / Storage | 0° to +50°C / -30° to +60°C |
| Temperature: | +32° to +122°F / -22° to +140°F |
| Connector type / position: | Type SMA Female / Side |
| Read distance: | 0 to 10 cm / 3.4 in. |

**Electrical Specifications**

| | |
|---|---|
| Frequency Range: | 864 - 928 MHz |
| Far-Field Gain: | -20dBi (greatly reduces stray tag-reads) |
| Typical VSWR across frequency range: | < 1.4 |
| Nominal Impedance: | 50 Ω |
| Maximum Input Power: | 1 W |

DOT 300 PDA reader

### Technical Specifications

Reading Range 0 - 50 cm
RFID (UHF) Compact 865 - 955MHz : ISO 18000-6C, EPC Class1 Gen2

### Scanning & Communications

1D Laser Scanner Barcode reader (optional) or 2D Imager (optional)
Memory ROM : NAND Flash, 512MB TSOP Option 256MB or 2GB

### Platform

AGX DOT 300
Keyboard 43 Qwerty oder 27 Numerisch
Windows Mobile 6.5 / Windows CE 5.0 but Android and iOS PDA's with UHF RFID also possible
RAM : mDDR, 256MB Option 128MB
4000mA for Compact / +2600mA for Gun version

### Power

### Power with RFID UHF

Standard 2400mA / Option 3600mA / Li-ion Polymer
Display 3.5" QVGA 240x320, VGA 480x640 260k color, touch
CPU Monahan PXA320 806MHz Prozessor

### Physical Charateristics

Standard 278g (incl. Standard battery) (DOT-300P 510g)

### Operating System

Dimensions 75.6mm * 25mm * 159mm / 174mm * 75mm * 129mm (DOT-300P) Weight

### Expansion Slot microSD Slot

Bluetooth Ver2.0 + EDR
3G Modem HSUPA/EDGE/GPRS/GSM Internal Antenna
WPAN (optional)
GPS (optional)
WWAN (optional)
Single slot cradle (Ethernet optional)

### Accessories

A-GPS with integrated antenna
Camera (optional) 3.0M pixel auto focus camera with LED flash
Communications USB 2.0
Reading Range 0 - 600 cm
Reading Range 0 - 6cm
RFID (HF) 13.56MHz : ISO14443A/B, ISO15693
CE, RoHs, KTL Drop Test 1.5m

### Wireless

### Others

### Cradle

Operating Temperature -10°C to 60°C
Storage Temperature -20°C to 70°C
WLAN (optional) IEEE 802.11 b,g Option CCX v4 Security (internal antenna)

### Environment

### Vehicle cradle

Leather case, Stylus pen
Adaptor, Screen protector, etc.

### Approvals

Humidity 5% - 95% non-condencing
Drop Drop test from 1.5m to concrete
Rain & Dust protection IP54

### Sato RFID Printer

### Printer and programmer for rfid labels

CL408e RFID and CL412e RFID High-Performance, High-Value Printers
For advanced technology and high-speed performance, the CLe Series printers have no match in the industry. Built in the tradition of SATO's rugged dependability, the CLe Series printers are based on RISC processors, delivering high-quality images at high speeds.
Supports EPC Class 0+, 1, Gen 2, I-Code, Tag-it
Industry Leading Throughput
203/305 dpi Print Resolution
4.1" Wide Print Area
New Generation RISC Microprocessor
Plug-In Interface Modules
RS232-57.6K bps
Parallel - IEEE1284

### Ethernet

Twinax/Coax
Downloaded TrueType Fonts
21 Barcode Symbologies (incl. 2-D)
CL408 and CL412 Compatible
Memory: 18 MB Standard and 20 MB Optional
M8485Se RFID High-Speed Print Engine

The world's largest supplier of OEM print engines, SATO continues to manufacture print engines that are regarded as the standard in the print/apply industry. Designed specifically for integration into automatic print/apply systems, the components of these high-duty cycle engines are selected for reliable performance over extended periods of time. They are available through (OEM) manufacturers of print/apply systems.
Supports EPC Class 1, Gen 2
Industry Leading Throughput
5" Wide Print Area
203 dpi Resolution
Up to 12 Inches/Second Print Speed
New Generation RISC Microprocessor
Plug-In Interface Modules
RS232-57.6K bps
Parallel - IEEE1284

### Ethernet

Twinax/Coax
Downloaded TrueType Fonts
21 Barcode Symbologies (incl. 2-D)
M8400S and M8485S compatible
Memory: 18 MB Standard and 20 MB Optional
Right and Left Hand Models Available

Medicinal products require controlled storage and transit conditions in order to ensure that their quality is not compromised. This applies to low-risk products as well as high-risk products such as vaccines, insulins and blood products (such as Factor VIII), which normally require storage between
2°C and 8°C. All distributors of drug products are required to record storage and transportation temperatures, as well as being licensed by the appropriate authorities. Temperature monitoring devices should be used to demonstrate compliance with the records that are kept. At every point in the chain, precautions should be taken to minimize the effect of external conditions on the quality and stability of the product.

It is mandatory that records should provide reliable up-to-date evidence of compliance, incase of audits and investigations from the MHRA and other stakeholders. Before setting up a storage facility, transport system or taking on a new range of products it is advisable that distributors carry out a risk analysis.

Currently, after production, medicine is sent to warehouse, with temperature on box level. Often there is no scanning on box level, and the trajectory from Pharmacy to patient, "The last mile", is not covered. Also, unused Medication, i.e. drugs that are not used in Pharmacy or at patient are destroyed because it is not known if temperature limits were exceeded! Furthermore, it cannot be ascertained if medication is taken by patient. A situation that is complicated by the fact that generally, from all the patients admitted in a hospital, only 7% has a correct medication file.

Regulatory Requirements in Healthcare
UDI (Unique Device Identification)
FMD (EU Falsified Medicine Directive)

### Deadlines:

9. February 2016*: requirements implementation in all EU Member States
* for Belgium, Italy and Greece: 9. February 2022
**Data Submission Portal:** Stakeholder model

### Delegated Acts on Safety Features:

- DataMatrix on Secondary level packaging

### The composition, format and carrier of the unique identifier will be fully harmonized across the EU

2011 EU Falsified Medications Directive 2D barcodes for prescription medicine
- Unique identification number, Batch/Lot number, Expiry date, Serial number, national reimbursement number (if applicable)

**4 or 5 Key** data element's : 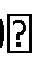

14 digit Manufacturer Product Code (GTIN in most markets) 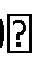

Randomized Unique Serial Number 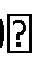 Expiry Date 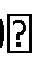

Batch Number - up to 20 alpha-numeric characters 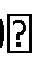

a national reimbursement number or other national number identifying, if required by the Member State

### Example:

**GTIN:** (01) 07046261398572
**Batch:** (10) TEST5632
**Expiry:** (17) 130331
**S/N:** (21) 19067811811

**Table 1 Examples of medication and temperature requirements at degrees Celsius (OC), and where applicable time requirements, in relation to administration.**

| | | |
|---|---|---|
| 1. | Hemophilus vaccine | + 2 to + 8 0C Single dose |
| 2. | 2. BCG Vaccine "N.B. Protect from light" | + 2 to + 8 0C 4 hours |
| 3. | 3. Di Te vaccine (Diphtheria Tetanus vaccine) | + 2 to + 8 0C One hour |
| 4. | 3. DPT vaccine (Diphtheria, Pertussis, Tetanus vaccine) | + 2 to + 8 0C One hour |
| 5. | 5. Hepatitis B vaccine | + 2 to + 8 0C Single dose |
| 6. | 6. Inactivated hepatitis A virus vaccine | + 2 to + 8 0C Single dose |
| 7. | 7. Inactivated split influenza vaccine | + 2 to + 8 0C Single dose |
| 8. | 8. Measles vaccine "N.B. Protect from light" | + 2 to + 8 0C Single dose |
| 9. | Meningococcal Meningitis type A&C vaccines. N.B. Protectfrom light. | + 2 to + 8 0C Three hours |
| 10. | MMR (Mumps, Measles, Rubella vaccine) + | + 2 to + 8 0C Single dose |
| 11. | Oral poliomyelitis vaccine (N.B. after thawing it can bekeptbetween +2 and +8 degrees 0C for six months). | - 20 to-26 0C Three hours |
| 12. | Rabies vaccine | + 2 to + 8 0C Single dose |
| 13. | Scorpion venom antitoxin | + 2 to + 8 0C Single dose |
| 14. | Snake venom antitoxin | + 2 to + 8 0C Single dose |
| 15. | Tetanus antitoxin (for passive immunization) | + 2 to + 8 0C Single dose |
| 16. | Tetanus toxoid for active immunization | + 2 to + 8 0C One hour |
| 17. | Typhoid vaccine (Typhim Vi) | + 2 to + 8 0C Single dose |

### Proposes system herein,

Assign a globally unique standardized identifier (the "UDI") to medical devices
- Place that "UDI" on the label / package / item in both plain human readable text (HRI) and also in an appropriate form or type of Automatic Identification and Data Capture (AIDC) data carrier
   - Apply "direct marking" for those devices which are intended to be reused or reprocessed
- Submit the required data related to product to a globally accessible database (e.g. US FDA's Global UDI Database or "GUDID")
- Follow through... IMPLEMENT for all medical devices as & when required!

In addition provide one of:
Open standard in CHIP: EPC-GENII
UHF RFID active label integrated in every item label.

### Temperature limits product:

Based on product ID barcode or data matrix, temperature limits are set or temperature limits can be preset in PDA scanner or in Cloud database.

### Battery Live:

If item is frozen, battery can be shut down
For long storage, battery can be shut down.
If battery is drained, energy is taken from reader (radiation from antenna).

### Safety:

If label is taken off, tamper in chip is broken.

Data in chip can be encrypted with password. Medicine that are not used in Pharmacy or at patient are destroyed because not sure if temperature limits were exceeded!

**Multiple read in 1 scan** instead of scan every item:
Multiple items are scanned before they are sent in box to warehouse. (UID)

### Extra information:

Every item location, data from label is send to Cloud (UID=> Cloud).

At every point in logistic chain, location is send, expiration date is checked and temperature limits are checked.

### Producer:

For recall, location of items is known.
Stock information, where are my articles?
Give client info when they run out of stock or there is lack of medication or vaccine, check where there is enough supply.

### Transporter:

Proof that transport conditions are met, for example for temperature, transport time etc.Check if goods are too long at customs out side fridge.

### Pharmacy:

Overview medication in stock
Overview medication over date
Check returned medication on re-use
Check medication against falsified database
Check stock
If medication is sold, change stocklevel.
If recall of medication check where item location is.

### Recall:

For a recall every location and item is known.

### Expiration date:

When expiration date is passed, a detailed report of all the items, location included can be generated (no search or manual check).

### Not used medication:

If medication is returned to Pharmacy, items can be checked on temperature and expiration date to check if they can be re-used.

### Counterfit drugs, falsified medication directive:

The data can be send and checked against national data base thur interface with Cloud.

### Check if patient has taken medication:

If the patient takes the medication, he can scan the medication.

Data is send to the National Falsified medication data base and item is deleted from database.

A report can be made with time and date to generate report of taken medication by patient.

## Claims

1. A method to survey handling of a medical or biological product said method comprising use of
a label for labelling a container suitable for containing said material, said label provided with information suitable for identifying said material, said label also provided with a monitoring device, and
a reader capable of providing a read-out of said monitoring device,
wherein
said monitoring device is provided with data suitable for identifying said material, and
said monitoring device is capable of recording handling conditions that may be detrimental to the quality of said material, and
said reader is capable of providing a warning signal when said handling conditions have been detrimental to the quality of said material.

2. A method according to claim 1 wherein said monitoring device comprises an electronic article surveillance (EAS) device and said reader is an EAS reader.

3. A method according to claim 1 or 2 wherein said monitoring device is a radiofrequency identification (RFID) device, preferably an active RFID device, and wherein said reader is an RFID reader.

4. A method according to claim 1 to 3 further comprising use of a writer capable of writing data in the monitoring device.

5. A method according to anyone of claims 1 to 4 wherein said reader is also capable of storing said data from said read-out in said monitoring device and/or in said reader and/or in an external data storing device such as an hard disk or a system of hard disks, preferably wherein said storing device is a cloud-based device.

6. A method according to anyone of claims 1 to 5 wherein said monitoring device is capable of recording handling conditions temperature and/or time.

7. A method according to anyone of claims 1 to 6 wherein said reader is capable of recording date of and/or time of and/or location of and/or identify a person taking part in providing said read-out.

8. A method according to anyone of claims 1 to 7 wherein said reader is capable of recording read-outs of at least two, preferably at least four, more preferably at least 10 monitoring devices that are simultaneously submitted to said reader.

9. A method according to anyone of claims 1 to 8 wherein said material comprises a medical product, such as a medicine, a drug, a vaccine, an hormone, an antibody, a protein or a peptide, or biological material such as blood, a blood component or a blood product.

10. A label for use in a method according to anyone of claims 1 to 9.

11. A label according to claim 10 comprising label information suitable for identifying a medical product or biological component and a monitoring device, such as an electronic article surveillance (EAS) device.

12. A label according to claims 10 or 11 further comprising means through which tampering with said label is made evident.

13. A label according to anyone of claims 10 to 12 wherein said label information comprises information encoded with a barcode.

14. A label according to anyone of claims 11 to 13 wherein said medical product is blood or a blood component.

15. A label according to claim 14 wherein said blood component is selected from the group of plasma, platelets or red blood cells.

16. A label according to any of claims 10 to 15 wherein said EAS device is a radiofrequency identification (RFID) device.

17. A label according to any of claims 10 to 16 wherein said EAS device is provided with an antenna and/or power source.

18. A label according to any of claims 10 to 17 wherein tampering results abolishing the function of said EAS device.

19. A label according to claim 18 wherein said tampering results in disrupting a connection with antenna or power source of said device.

20. A label according to any of claims 11 to 19 wherein said monitoring device allows for temperature sensing of the content of said container.

21. A label according to claim 20 wherein said monitoring device allows for tracking and tracing said container.

22. A method to control quality of a medical or biological product, such as a medicine, a drug, a vaccine, an hormone, an antibody, a protein or a peptide, or a blood product, a cell or cells, a tissue or an organ, comprising using a label according to any of claims 10 to 21 to label the container in which said product is stored or transported.

23. A method to track and trace a medical or biological product, such as a medicine, a drug, a vaccine, an hormone, an antibody, a protein or a peptide, or a blood product, a cell or cells, a tissue or an organ, comprising using a label according to any of claims 10 to 21 to label the container in which said product is stored or transported.

24. A method for providing a chain of documentation arising from the chain of events from the donation of donor blood to the transfusion of a blood product to a recipient comprising labelling a container comprising blood or a blood component with a label according to any of claims 10 to 21.

25. A method according to any of claims 22 to 24 comprising using at least a second integrated label after a processing step in the processing of said medical or biological product.

26. A method according to claim 25 wherein said processing step comprises centrifugation or splitting of a blood product.

27. A system to control quality of a medical product, such as a blood product, a cell or cells, a tissue or an organ, comprising using a label according to any of claims 10 to 21 to label the container in which said product is stored or transported.

28. A system to track and trace a medical product, such as a blood product, a cell or cells, a tissue or an organ, comprising using a label according to any of claims 10 to 21 to label the container in which said product is stored or transported.

29. A system according to any of claims 27 or 28 comprising using at least a second label after a processing step in the processing of said biological product and reading data from the log file of a first integrated label and importing said date into the log file of said second integrated label.

30. A system according to claim 29 wherein said processing step comprises centrifugation or splitting of a blood product.

31. A container such as a blood bag provided with a label according to any of claims 10 to 21.

32. Use of a label according to any of claims 10 to 21 or a method according to any of claims 1 to 9 or 22 to 26 or a system according to any of claims 27 to 30 or a container according to claim 31 to prevent a receptor from receiving a medical product from a non-intended or wrong source and/or to collect haemovigilance data.
